# EUROPEAN PATENT SPECIFICATION

(11) **EP 1 765 989 B1**
(45) Date of publication and mention of the grant of the patent: **02.11.2016**
(21) Application number: 05804866.1
(22) Date of filing: 02.06.2005
(51) Int. Cl.: C12N 5/16, C12N 5/079, C12N 5/0797, A61K 35/12, A61K 48/00, A01K 67/027

(54) **THERAPEUTIC DELIVERY OF ADENOSINE INTO A TISSUE**
THERAPEUTISCHE ABGABE VON ADENOSIN IN EIN GEWEBE
ADMINISTRATION THERAPEUTIQUE D'ADENOSINE DANS UN TISSU

(30) Priority: 02.06.2004 US 858929
(43) Date of publication of application: 28.03.2007
(73) Proprietor: Life & Brain GmbH, 53105 Bonn (DE)
(72) Inventor: BRUSTLE, Oliver, Institute of Reconstructive, Sigmund -Freud-Strasse 25 53105 Bonn (DE); KOCH, Peter, Life & Brain GmbH, 53105 Bonn (DE); MOHLER, Hanns, CH-8706 Feldmeilen (CH); BOISON, Detlev, CH-8050 Zurich (CH)
(74) Representative: Grund, Martin
(86) International application number: PCT/US2005/019280
(87) International publication number: WO 2005/118789

(56) References cited:
- WO-A2-03/046141
- US-A- 6 110 902
- FEDELE D E ET AL: "Engineering embryonic stem cell derived glia for adenosine delivery" NEUROSCIENCE LETTERS, LIMERICK, IE, vol. 370, no. 2-3, 11 November 2004 (2004-11-11), pages 160-165, XP004607325 ISSN: 0304-3940
- GUTTINGER M ET AL: "Suppression of kindled seizures by paracrine adenosine release from stem cell-derived brain implants" EPILEPSIA 2005 UNITED STATES, vol. 46, no. 8, 2005, pages 1162-1169, XP002438790 ISSN: 0013-9580 1528-1167
- ZUMSTEG V. ET AL: 'The use of real-time PCR with fluorogenic probes for the rapid selection of mutant neuroectodermal grafts' JOURNAL OF NEUROSCIENCE METHODS vol. 120, no. 1, October 2002, pages 85 - 94, XP002996051
- BOISON D. ET AL: 'Neonatal hepatic steatosis by disruption of the adenosine kinase gene' PROCEEDINGS OF THE NATIONAL ACADEMY OF SCIENCES USA vol. 99, no. 10, 14 May 2002, pages 6985 - 6990, XP002996052
- MORTENSEN R.M. ET AL: 'Production of Homozygous Mutant ES Cells with a Single Targeting Construct' MOLECULAR AND CELLULAR BIOLOGY vol. 12, no. 5, May 1992, pages 2391 - 2395, XP002080094
- LANG K.J.D. ET AL: 'Differentiation of Embryonic Stem Cells to a Neural Fate: A Route to Re-building the Nervous System' JOURNAL OF NEUROSCIENCE RESEARCH vol. 76, 15 March 2004, pages 184 - 192, XP008030579
- LANDRY DONALD W ET AL: "Embryonic death and the creation of human embryonic stem cells", November 2004 (2004-11), JOURNAL OF CLINICAL INVESTIGATION, VOL. 114, NR. 9, PAGE(S) 1184-1186 ISSN: 0021-9738
- VRANA KENT E ET AL: "Nonhuman primate parthenogenetic stem cells.", 30 September 2003 (2003-09-30), PROCEEDINGS OF THE NATIONAL ACADEMY OF SCIENCES OF THE UNITED STATES OF AMERICA, VOL. 100, NR. SUPPLEMENT 1, PAGE(S) 11911-11916 ISSN: 0027-8424
- LAVERGE H ET AL: "Fluorescent in-situ hybridization on human embryos showing cleavage arrest after freezing and thawing", February 1998 (1998-02), HUMAN REPRODUCTION (OXFORD), VOL. 13, NR. 2, PAGE(S) 425-429 ISSN: 0268-1161
- ALIKANI MINA ET AL: "Human blastocysts from aggregated mononucleated cells of two or more non-viable zygote-derived embryos.", July 2002 (2002-07), REPRODUCTIVE BIOMEDICINE ONLINE 2002 JUL-AUG, VOL. 5, NR. 1, PAGE(S) 56 - 58 ISSN: 1472-6483
- Judgement C-34/10 of the European Court of Justice
- Judgement X ZR 58/07 of the European Court of Justice
- Judgement C-364/13 of the European Court of Justice

<110> Life & Brain GmbH
   Koch, Peter
   Brustle, Oliver
   Mohler, Hanns
   Boison, Detlev
<120> THERAPEUTIC DELIVERY OF ADENOSINE INTO A TISSUE
<130> 16052/2008
<140>
   <141> 2005-06-02
<160> 3
<170> PatentIn version 3.2
<210> 1
   <211> 27
   <212> DNA
   <213> Artificial Sequence
<220>
   <223> sense primer specific for wild-type Adk
<400> 1
   ctcacttaag ctgtatggag gtgaccg 27
<210> 2
   <211> 27
   <212> DNA
   <213> Artificial Sequence
<220>
   <223> antisense primer specific for wild-type Adk
<400> 2
   agtcacagat gcatctgcag aggtgag 27
<210> 3
   <211> 26
   <212> DNA
   <213> Artificial sequence
<220>
   <223> antisense primer specific for targeting construct
<400> 3
   actgggtgct caggtagtgg ttgtcg 26

## Description

### FIELD OF THE INVENTION

The present invention relates to the treatment of conditions associated with neuronal activity. In particular, the present invention relates to compositions and methods for therapeutic delivery of adenosine to treat neuronal disorders in a subject.

### BACKGROUND OF THE INVENTION

Epilepsy is a relatively common neurological disorder with a lifetime prevalence of about 2 to 5% that manifests itself in varied forms of epileptic seizures. These seizures range from brief lapses of attention (absence seizures) to limited motor, sensory or psychological changes (partial seizures) to prolonged losses of consciousness with convulsive motor activity (idiopathic or symptomatic generalized tonic clonic seizures). Such symptoms are due to synchronous discharges of large populations of neurons based on a deficiency in inhibitory neurotransmission or an excess of excitatory neurotransmission. Current drug therapy cannot completely suppress seizure occurrence in approximately 60% of patients (McNamara, 1994, J. Neurosci. 14:3413-3425). Additionally, therapy is often accompanied by adverse drug effects. Drug resistant forms of epilepsy (most of them mesial temporal lobe epilepsies) require resective surgery of the epileptogenic focus. For many patients, this kind of intervention is a final option that carries an inherent risk of morbidity. Even after surgery, most epileptic patients continue to require antiepileptic drug medication for many years.

One potential therapeutic compound for inhibiting epileptic activity and pain is adenosine. Adenosine is an endogenous compound with known chemical structure. It occurs naturally in low concentrations in nearly all cells of the body but is normally not released except in some pathological conditions. When adenosine is applied in pharmacological doses to various organ *systems in vitro* it exerts multiple effects by acting on adenosine receptors (A₁ and A₂). Relatively low systemic doses of adenosine receptor agonists produce marked sedation and hypothermia. High doses of adenosine reportedly cause cessation of spontaneous motor activity as well as some ataxia (Dunwiddie and Worth, 1982, J. Pharmacol. Exp. Therap. 220:70-76).

Adenosine is a potent inhibitory neuromodulator of the brain (Dunwiddie and Masino, 2001). In brain tissue *in vitro*, application of adenosine strongly inhibits neuronal activity (Guieu et al., 1996, Clinical Neuropharmacology 19, 459-474). Adenosine specifically and potently reduces neuronal excitation by inhibiting the release of excitatory neurotransmitters such as glutamate in a presynaptic, calcium dependent mechanism (Thomson et al., 1993, TINS 16:222-227; Wu and Saggau, 1994, Neuron 12:1139-1148). This effect is mediated via activation of the adenosine A₁-receptors (Fredholm, 1995, NIPS 10:122-128). In humans, microdialysis studies demonstrated a release of adenosine during seizure activity and adenosine was proposed to be a natural mediator of seizure arrest and postictal refractoriness (During and Spencer, 1992). Pharmacologically, adenosine and its analogues acting on adenosine A₁ receptors have potent inhibitory effects on neuronal activity, and are reportedly effective in seizure suppression and neuroprotection (Boison et al., 2002a; Boison et al., 1999; Fredholm, 1997; Huber et al., 2002; Huber et al., 2001). Of particular relevance was the recent finding that activation of adenosine A₁ receptors led to the suppression of seizures in a model of pharmacoresistant epilepsy, thus pointing to adenosine as a potential therapeutic advance compared to conventional antiepileptic drugs (Gouder et al., 2003).

The accumulation and release of adenosine is mainly controlled by the activity of adenosine kinase (commonly referred to as "ADK"; EC 2.7.1.20), the key enzyme of adenosine metabolism. This notion is based on several lines of evidence: (i) pharmacological inhibition of ADK in hippocampal slices provides adenosine A₁-receptor mediated presynaptic inhibition (Pak et al., 1994); *ii)* pharmacological inhibition of ADK provides seizure suppression in various models of epilepsy (Kowaluk and Jarvis, 2000); and *(iii)* certain types of cells have a high flux rate in a futile cycle involving ADK and 5'-nucleotidase (Bontemps et al., 1983). As a consequence, inhibition of ADK potentially leads to rapid and large increases in adenosine. Conversely, over-expression of ADK has been associated with epileptogenesis, thus providing a rationale for adenosine-mediated therapeutic intervention (Gouder et al., 2004).

Adenosine and its analogues have powerful anti-seizure activities in various models of epilepsy. However, when administered systemically, adenosine and its analogues cause strong, adverse effects ranging from sedation and hypothermia to the suppression of cardiovascular functions and an almost complete cessation of spontaneous motor activity, preventing its therapeutic use (Dunwiddie, TV, 1999, Adv. Neurol. 79: 1001-1010). Moreover, although several research groups report a temporary protective effect of adenosine against epileptic activity, none have achieved true long-term efficacy.

### SUMMARY OF THE INVENTION

[ ]₁ The present invention relates to generation of embryonic stem (ES) cells and neural cells having deficient adenosine kinase gene on both alleles. The present invention further relates to a composition comprising the generated neural cells suitable for implantation into a mammal. The present invention further relates to the delivery of sustained therapeutic doses of adenosine to a subject for treatment of disorders related to neural activities in the subject. Preferably the disorders include epilepsy and chronic neural pain.

One aspect of the present disclosure is directed to neural cells differentiated from ES cells with the defect in both alleles of adenosine kinase gene. The differentiated neural cells may be neural precursor cells or mature neural cells such as glial cells, all with the defect of adenosine kinase gene in both alleles. Furthermore, the differentiated neural cells release a therapeutic amount of adenosine both *in vitro* and in a host subject.

Disclosed are methods of chronic local delivery of the genetically engineered cells to a tissue site of a subject associated with neuronal activity. Preferably, the neuronal activity is epileptic activity. In one embodiment, the genetically engineered cells are encapsulated into a semipermeable polymer membrane and transplanted into a tissue site of a host subject. In another embodiment, the cells are directly transplanted into the target area of a tissue site. Preferably, the cells transplanted are neural precursor cells. More preferably, the neural precursor cells have a deficient ADK gene on both alleles. Preferably, the neural precursor cells are transplanted into central nervous system such as brain.

Further disclosed is a method of treating neural disorders associated with neuronal activity by using cell-based local delivery of therapeutic compositions and cells. Preferably, disclosed is a method of treating epilepsy. Preferably, the method supplies a local chronic dose of adenosine to treat epilepsy by implanting into the epileptogenic focus adenosine-releasing cells or adenosine-releasing cells embedded in a polymer membrane. More preferably, the dose is in the rate of about 1 ng to about 500 ng of adenosine per day. Also preferably, the adenosine-releasing cells have a defect of adenosine kinase gene in both alleles.

The invention is defined by the appended claims.

The invention provides an isolated human or mouse neural precursor cell deficient in the production of adenosine kinase, said cell comprising a defect in both alleles of the adenosine kinase gene, wherein said cell is able to differentiate into a neuron or glial cell, obtainable by treating embryonic stem (ES) cells which have been isolated from a mammal, (a) so as to disrupt one allele of the adenosine kinase gene in said ES cell, thereby deriving an ES cell comprising a defect in one allele of an adenosine kinase gene; treating said ES cell derived from step (a) so as to (b) inactivate the remaining allele of adenosine kinase gene, thereby generating an ES cell comprising a defect in both alleles of the adenosine kinase gene; and differentiating said ES cells generated in step (b) to a neural precursor cell.

Disclosed is also a composition comprising the neural cells of the present invention and a polymer suitable for implantation into a mammal. Preferably, the composition contains between about 20,000 to about 2,000,000 of the neural cells. More preferably, the composition contains 50,000, 100,000, 500,000, or 1,000,000 cells.

Further disclosed is a method of producing a neural precursor cell of the present invention. The method includes first producing an ES cell having disrupted adenosine kinase gene in both alleles and differentiating the ES cells into the neural precursor cell of the present invention.

### BRIEF DESCRIPTION OF THE FIGURES

Figures not pertaining to the invention are for illustrative purposes only.

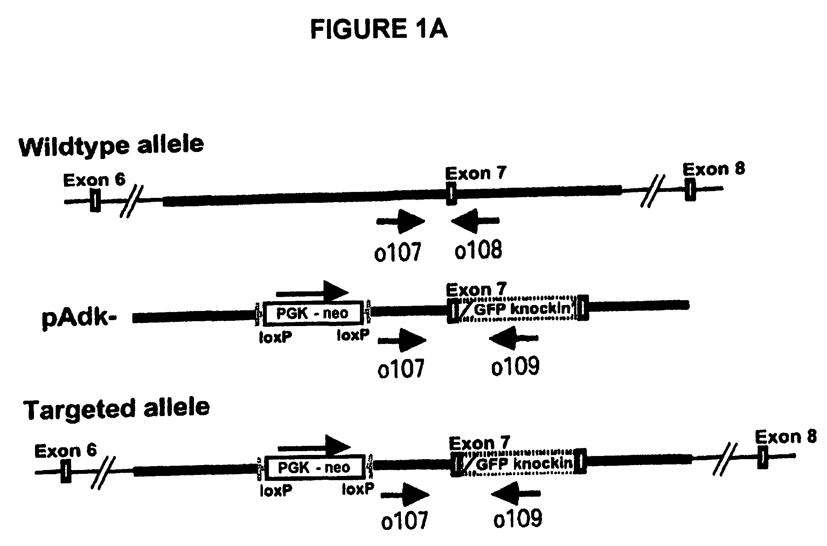

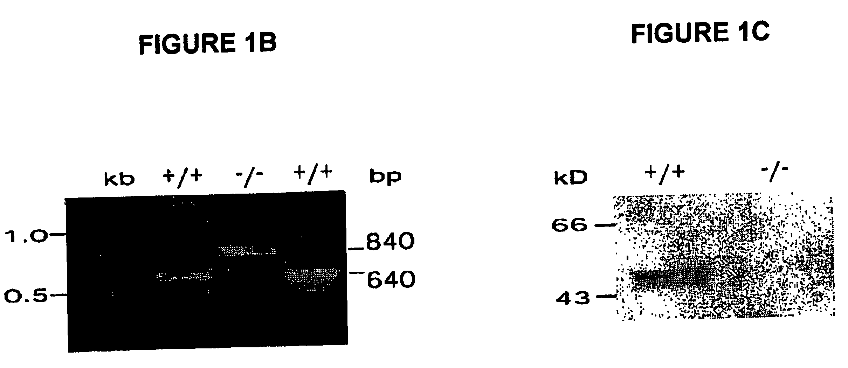

Figure 1 shows bi-allelic homologous recombination of the murine *Adk* locus. *a*. Gene maps of the murine *Adk* wild-type allele, targeting vector pAdk-, and targeted allele. The thick lines indicate genomic sequences of the 400kb murine *Adk* gene homologous to the targeting vector, while thin lines indicate external *Adk* sequences not present in the targeting vector. *Adk*-exons 6, 7, and 8, as well as the insertion cassette for the neomycin resistance gene (PGK-neo), are outlined as open boxes, loxP sites as vertical bars. PGK-neo is transcribed in the direction indicated by the arrow. The GFP-knock-in cassette (hatched box) for the enhanced green fluorescent protein is fused in-frame to *Adk*-specific exon 7, thus causing a disruption of the *Adk* gene. The black arrows indicate the location of oligonucleotide primers o107, o108, and o109 used for PCR analysis. *b*, PCR analysis of the murine *Adk* locus. An ES cell clone lacking one Adk allele was previously generated by gene targeting with the pAdk-vector (Boison et al., 2002b). To achieve a bi-allelic knockout of *Adk* cells from this clone were transfected again with the same vector and selected with MMPR for ADK-deficiency. Genomic DNA was amplified using a combination of the three allele specific primers o107, o108, and o109. DNA derived from wild-type ES cells (+/+) gives rise to a 640 bp band (primers o107/o108), while DNA derived from an MMPR resistant clone gives rise only to the 840 bp band (primers o107/o109) being indicative for a bi-allelic genetic disruption of the *Adk* locus. The DNA size marker shown is a 1 kb-ladder (Life Technologies). c, Western Blot analysis of cell extracts from *Adk*^{+/+} (+/+) and *Adk*^{-/-} (-/-) ES cells probed with a polyclonal rabbit antiserum raised against recombinant mouse ADK. In samples taken from wild-type cells (+/+) a double band was detected in the size range of 44 to 46 kDa. No ADK specific protein was detected in samples derived from MMPR-resistant *Adk*^{-/-}-cells (-/-).

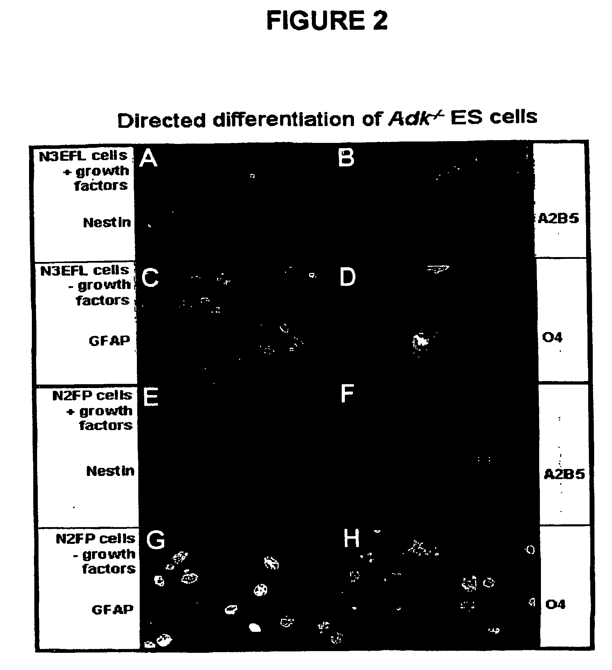

Figure 2 shows differentiation of Adk-deficient ES cells into glial cells. ES cell-derived glial precursors proliferating in FGF2/EGF (N3EFL PROL) and FGF2/PDGF (N2FP PROL) were induced to differentiate by a 4 day growth factor withdrawal (N3EFL DIFF; N2FP DIFF). During proliferation, both FGF2/EGF- and FGF2/PDGF-dependent precursors express nestin and A2B5. Following growth factor withdrawal, they generate GFAP-positive astrocytes and 04-immunoreactive oligodendrocytes. No obvious differences in glial differentiation were noted between *Adk*^{-/-} and *Adk*^{+/+} ES cells.

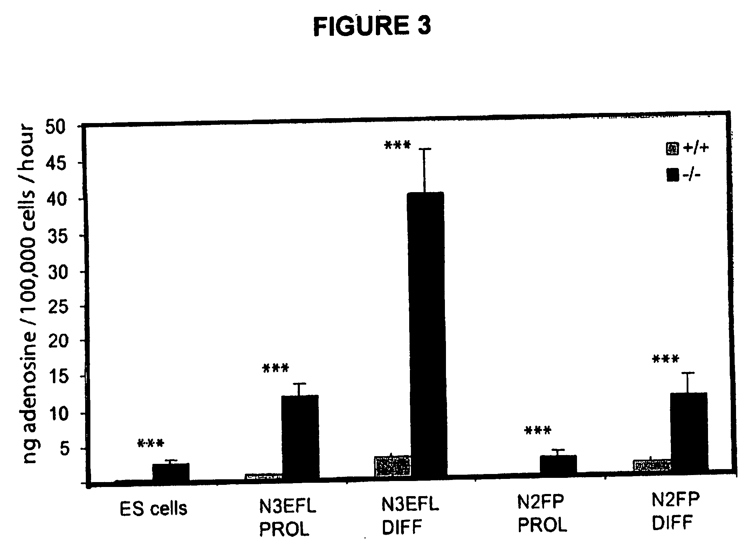

Figure 3 shows that adenosine is released from *Adk*^{*-*/*-*} ES cells and their glial progeny. Supernatant samples from the medium of ES cell-derived replicate cultures (n = 6, each) were analyzed for adenosine content. These values were normalized to the number of cells and are represented as adenosine released per 10⁵ cells per hour. Samples were taken from wild-type (gray bars, +/+) or *Adk*^{-/-} (black bars, -/-) undifferentiated ES cells, FGF2/EGF-dependent glial precursors (N3EFL) and FGF2/PDGF-dependent glial precursors (N2FP). Glial precursors were studied both during proliferation (PROL) and following growth factor withdrawal-induced differentiation (DIFF). Errors are given as ± SD. Significance values have been calculated by unpaired t-test, *** = p<0.0001.

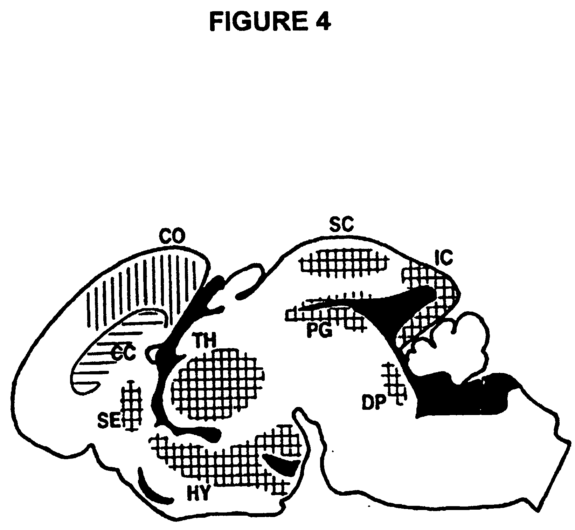

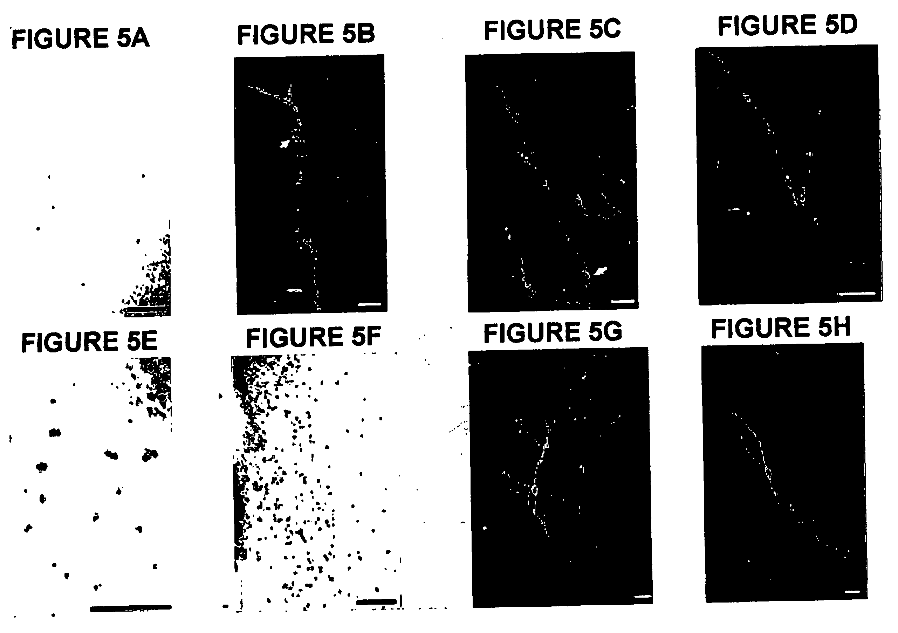

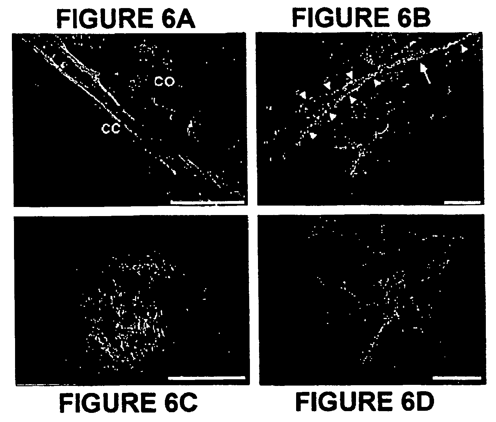

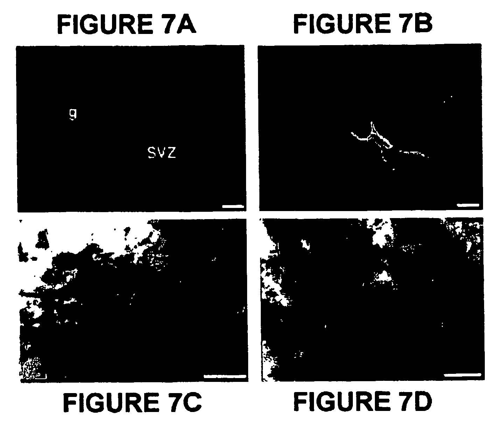

Figure 4 shows integration and distribution of ES-cell-derived neural precursors after implantation into the telencephalic vesicle of E16-E18 rats. The schematic represents a midsagittal section through the brain of a newborn recipient. After leaving the ventricular system (solid areas), neurons (vertical lines) and astrocytes (horizontal lines) occupy overlying territories. Donor-derived neurons integrate preferentially into gray matter regions exhibiting neurogenesis until or beyond the time of implantation. ES-cell-derived astrocytes also incorporate into white matter regions such as the corpus callosum (CC). CO, cortex; DP, dorsal pontine area; HY, hypothalamus; IC, inferior colliculus; PG, periaqueductal gray; SC, superior colliculus; SE, septum; TH, thalamus. Donor-derived neurons and astrocytes were also detected in hippocampus (Fig. 6*B*), olfactory bulb (Fig. *7A* and *7B*), and striatum (Fig. *5E*).

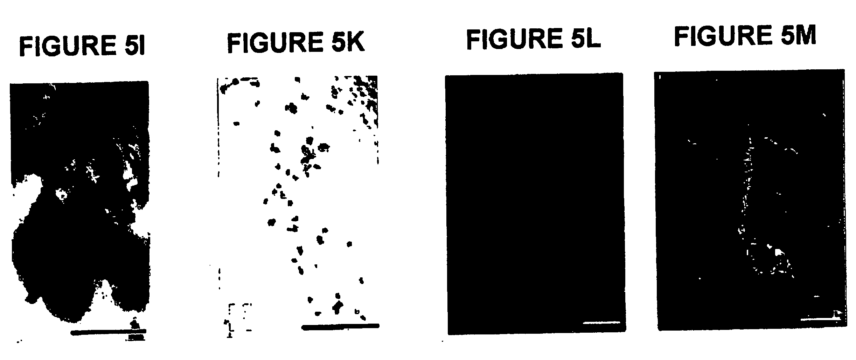

Figure 5 shows that ES-cell-derived neural precursors injected into the telencephalic vesicle of fetal rats incorporate individually into a variety of host brain regions and differentiate into neurons. Donor cells are identified by *in situ* hybridization using a digoxigenin-labeled probe to mouse satellite DNA (*A*, *E, F,* and *I-L*). Immunofluorescence detection of the mouse-specific antigen M6 and confocal laser microscopy were used to reconstruct individual neuronal profiles (*B-D*, *G, H,* and *M*). (*A-D*) Six days after injection into the telencephalic vesicle of an E17 rat, donor cells have left the ventricle and incorporated into the host cortex. The ES-cell-derived neurons show prominent apical dendrites and basal axons entering the corpus callosum, a morphology appropriate for cortical projection neurons (arrows: perikaryon). Note the characteristic pyramidal morphology in *D. (E)* Incorporated donor cells in the striatum of a 2-week-old rat transplanted at E18. (*F*-*H*) Donor-derived cells in the host hypothalamus. In contrast to cortex, neurons incorporating into the diencephalon frequently exhibited multipolar morphologies (G and *H*). (*I*) Host- and donor-derived neurons in the septum of a newborn rat. Both neurons show expression of microtubule-associated protein 2; the donor-derived cell is identified by *in situ* hybridization. (*K*-*M*) Incorporated cells in the host thalamus of newborn *(K)* and 2-week-old rats *(L* and *M*). In *L*, ES-cell-derived neurons are visualized by fluorescence *in situ* hybridization (green dots) and subsequent immunofluorescence analysis with an antibody to the nuclear neuronal antigen NeuN (red). Note the mature neuronal phenotype of the integrated cells with the presence of dendritic spines (M). [Bars = 100 µm *(A, E, F*, and *K*), 20 µm (*B*-*D*, *G, H*, *L,* and *M*), and 10 µm (*I*).]
Figure 6 shows extensive axonal innervation of the host brain. The ES-cell-derived neurons generated a dense axonal network within the recipient brains. Abundant M6-positive axons were found at all levels in both gray and white matter. (*A*) Donor-derived axons in corpus callosum (cc) and deep layer cortex (co) of a 2-week-old recipient. (B) Axonal innervation of the hippocampal stratum oriens. The M6 immunofluorescence also depicts the perikaryon (arrow) and dendrites (arrowheads) of a large horizontal neuron in the upper stratum oriens. The morphology of this cell is very similar to the outline of Golgi-impregnated horizontal neurons in this area. (C) Abundant donor-derived axons in a striatal fiber tract of 2-week-old recipient brain. (D) ES-cell-derived axons in the thalamus of a newborn recipient transplanted at E17. (Bars = 50 µm.)
Figure 7 shows incorporation of ES-cell-derived glia. (*A* and *B*) into the host. ES-cell-derived astrocytes have migrated into the granular layer (g) of the olfactory bulb of a 2-week-old host. Cells are visualized with an antibody to the mouse-specific antigen M2 (red). SVZ, olfactory subventricular zone. An individual astrocyte, double labeled with an antibody to GFAP (green), is shown in *B.* (C *and D)* ES-cell-derived oligodendrocytes in the rostral (C) and caudal (D) corpus callosum of a 2-week-old host brain. The donor cells, identified by DNA *in situ* hybridization (black), are morphologically indistinguishable from adjacent host oligodendrocytes. Host- and donor-derived oligodendrocytes exhibit equivalent immunoreactivity to CNPase (red). [Bars = 100 µm *(A)* and 10 µm (*B-D*).]

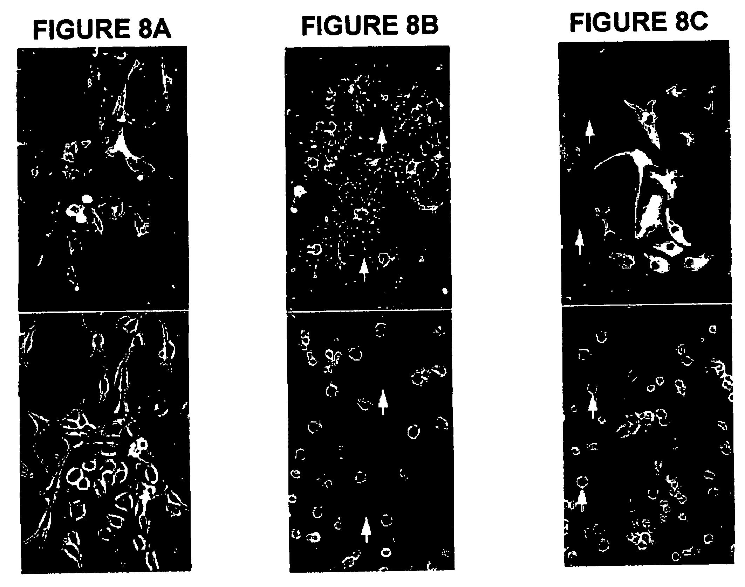

Figure 8 shows morphology and antigen expression of ES cell-derived glial precursors. (A) Cells grown in the presence of FGF2 and PDGF show immunoreactivity with the A2B5 antibody. (B) Four days after growth factor withdrawal, many cells assumed the typical multipolar morphology of oligodendrocytes and express the oligodendroglial antigen 04. Note the unlabeled cells with a flat, astrocytic phenotype (arrows). (C) The same culture contains numerous astrocytes expressing the astrocyte-specific intermediate filament GFAP. Note the unlabeled cells with multipolar oligodendroglial morphology (arrows). Immunofluorescent and phase contrast pictures are shown in the upper and lower panels, respectively.

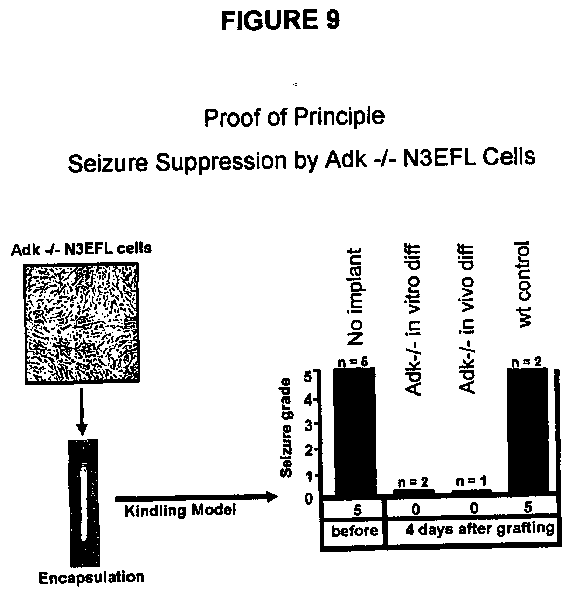

Figure 9 shows seizure suppression by ADK-/- N3EFL cells. Schematic representation of the experimental paradigm used for seizure suppression by transplanted ADK-/- N3EFL cells. ADK-/- ES cells were differentiated to glial precursors growing in the presence of FGF2 and EGF according to Brüstle et al., Science, 285: 754-756 (1999). In vitro differentiation of N3EFL cells was induced by a 3 day growth factor withdrawal. 150'000 proliferating and differentiated cells each were encapsulated into individual preformed polyethersulfone (PES) polymer hollow-fiber capsules containing a polyvivylalcohol (PVA) matrix (7-mm long, 0.5-mm inner diameter, wall thickness of 50 µm) as described (Huber, A. et al., 2001, PNAS USA, 98(13):7611-7616). Before experimental use the cell capsules were kept *in vitro* in the presence of FGF2 and NGF for a total of 7 days, except for the *in vitro* differentiation, were growth factors were omitted during the last 3 days. Rats were subjected to kindling as described (Huber, A. et al., 2001, PNAS USA, 98(13):7611-7616). After completion of kindling, capsules containing either proliferating (n = 1) or differentiated (n = 2) ADK-/- N3EFL cells or proliferating wild type N3EFL control cells (n = 2) were implanted into the lateral ventricle ipsilateral to the site of stimulation. Seizure activity was evaluated before and 4 days after implantation. As shown in the bar graph, recipients engrafted with differentiated (Adk-/- in vitro diff) and proliferating (Adk-/- in vivo diff) N3EFL cells displayed a complete suppression of seizure activity. Implantation and seizure evaluation were performed according to Huber, A. et al., 2001, PNAS USA, 98(13):7611-7616.

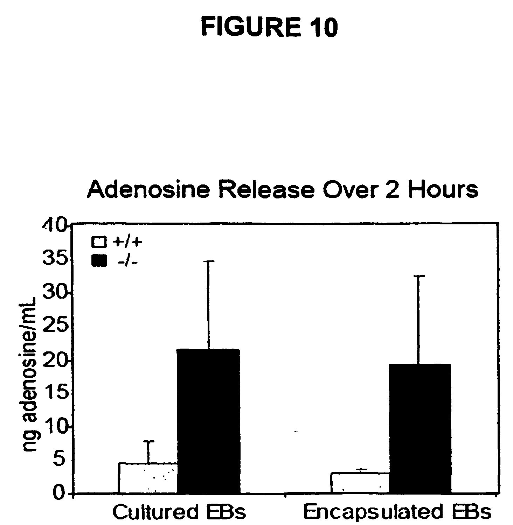

Figure 10 shows adenosine release from cultured and encapsulated Ebs. Error bars: ±SD.

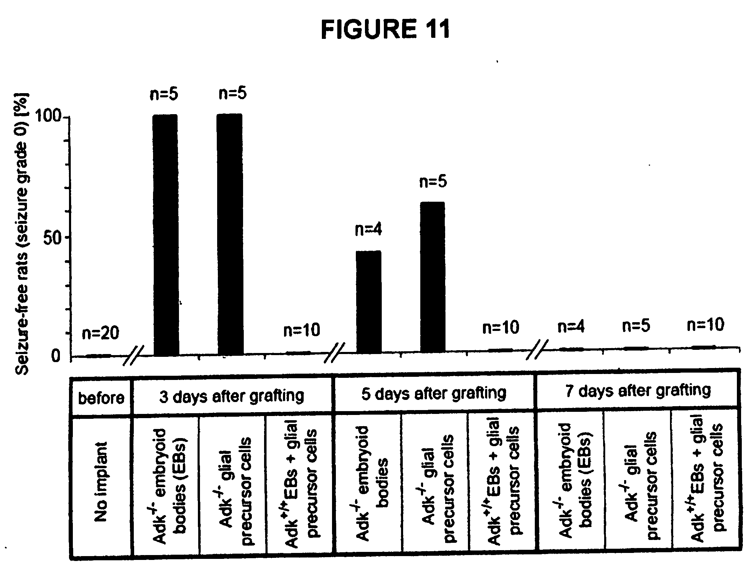

Figure 11 shows seizure suppression by *Adk*^{-/-} ES cell-derived implants.

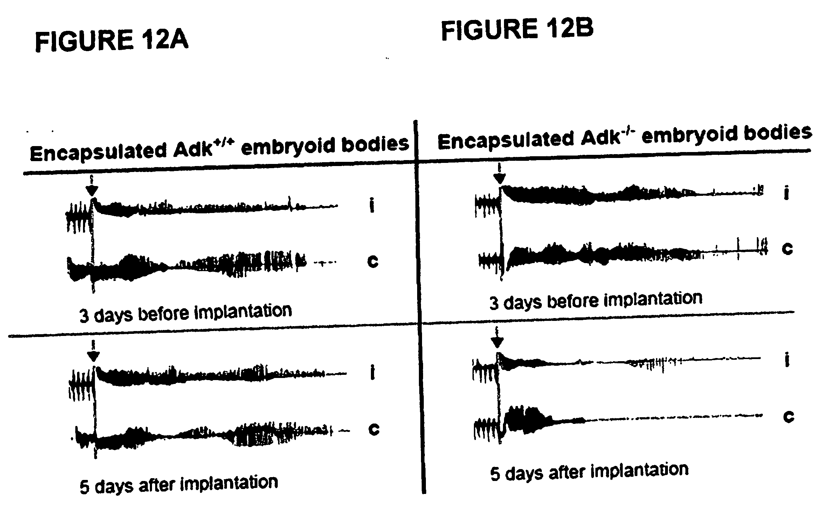

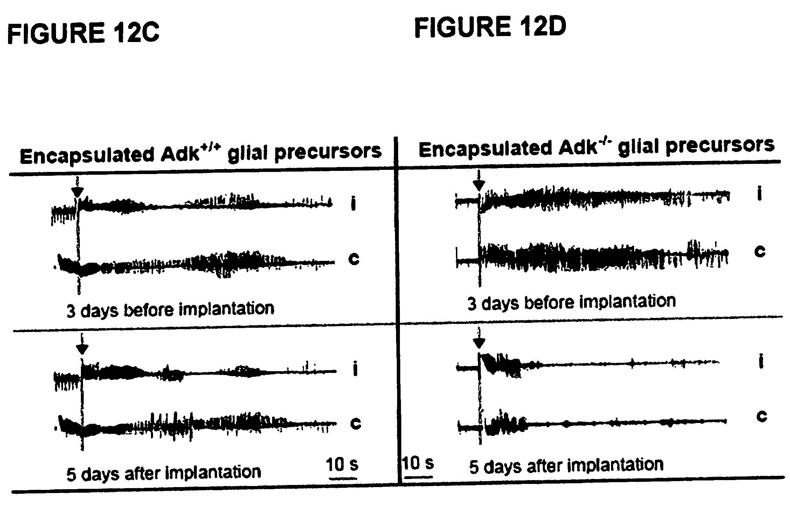

Figure 12 shows representative EEG recordings after application of test stimulations in kindled rats. Bilateral intrahippocampal EEGs were recorded after application of a test stimulus in kindled rats that had received (Panel A) a control *Adk*^{+l+} or (Panel B) an adenosine-releasing *Adk*^{-/-} EB capsule or (Panel C) a control *Adk*^{+/+} or (Panel D) an adenosine-releasing *Adk*^{-/-} glial precursor cell capsule.

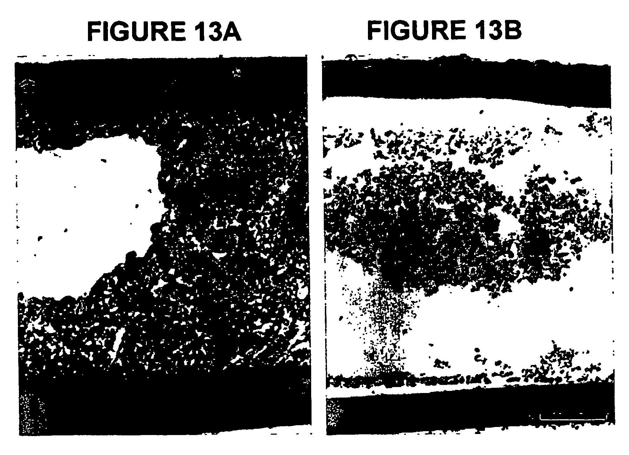

Figure 13 shows hematoxylin-eosin staining of encapsulated ES cell-derived *Adk*^{-/-} EBs. Panel (A) shows a representative capsue retrieved 2 days after implantation which shows purple-stained nuclei and pink-stained cytoplasm of viable cells within the capsule. Panel (B) shows a representative capsule retrieved 7 days after implantation which shows evidence of cells that have been loaded into the capsule, but none remain viable at this time point.

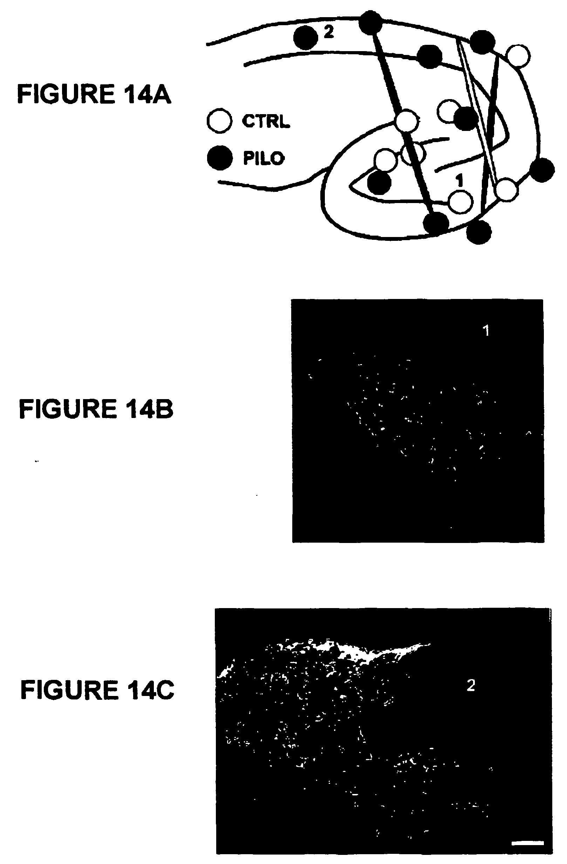

Figure 14 shows a shematic and micrograph of transplant morphology and localization. Panel A shows the cites of implantation. Circles: clusters; Rods: injection canals. The numbers correspond to the transplant depicted in Panels B and C. Panels B and C show the visualization of transplants with double immunofluorescence staining for both EGFP and M6, a moust specific membrane-bound protein (B: control; C: pilocarpine-treated; scale bar = 100µm).

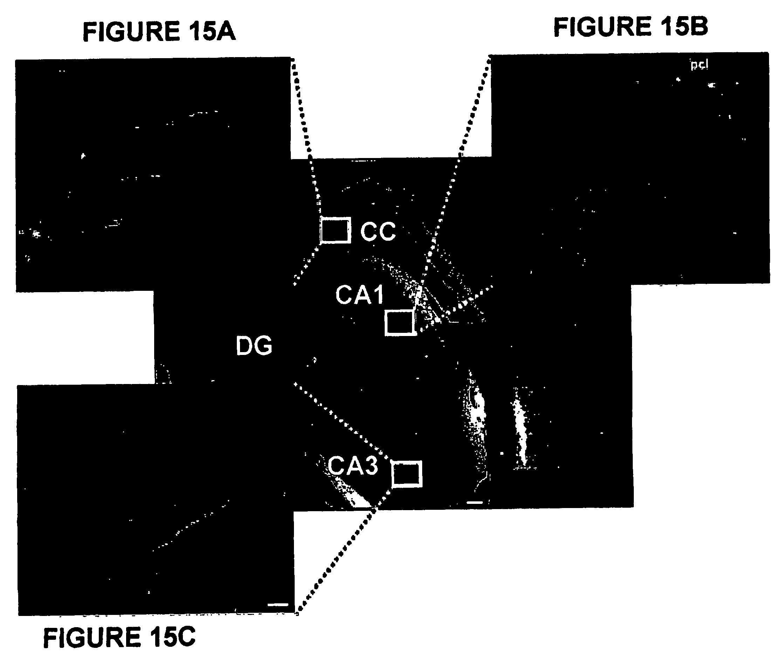

Figure 15 shows photomicrographs of an experiment in which transplantation was carried out into a pilocarpine-treated rat. The central portion of the figure depicts the location of the transplant. Overlay of phase contrast (black and white), and immunofluorescence with antibodies to M6 (red) and EGFP (green) (scale bar corresponds to 200 µm). Squares indicate location of closeup photomicrographs in the corpus callosum (A), CA1 region (B) and CA3 region (C, scale bars correspond to 25 µm). The pyramidal region of CA1 in pilocarpine-treated animals shows a pronounced autofluorescence unrelated to the EGFP signal (see panel B). Abbreviations: pcl, pyramidal cell layer; cc, corpus callosum.

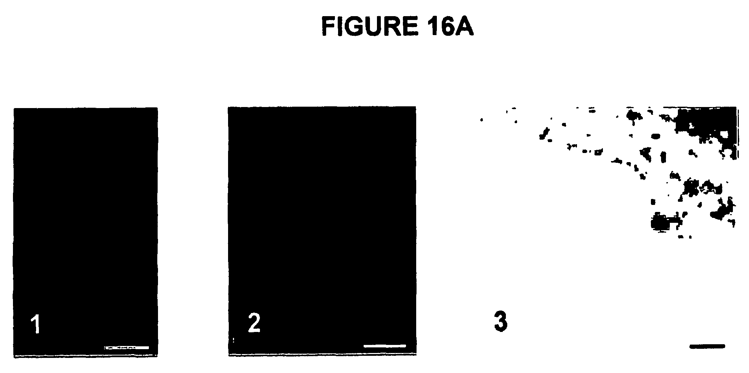

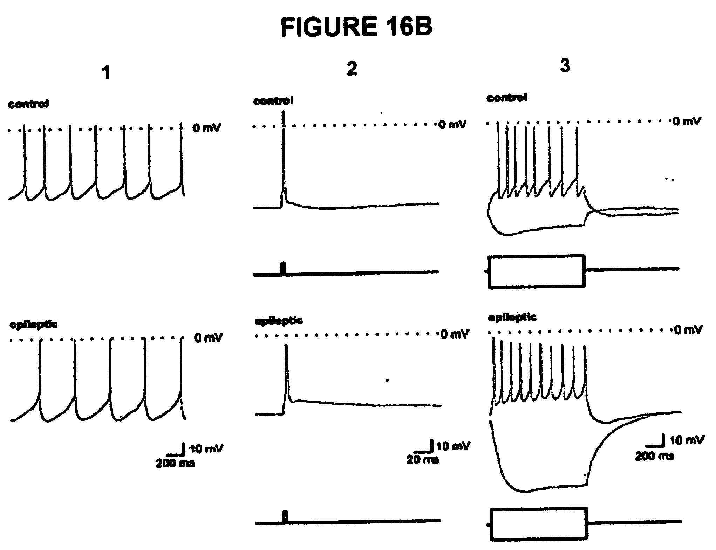

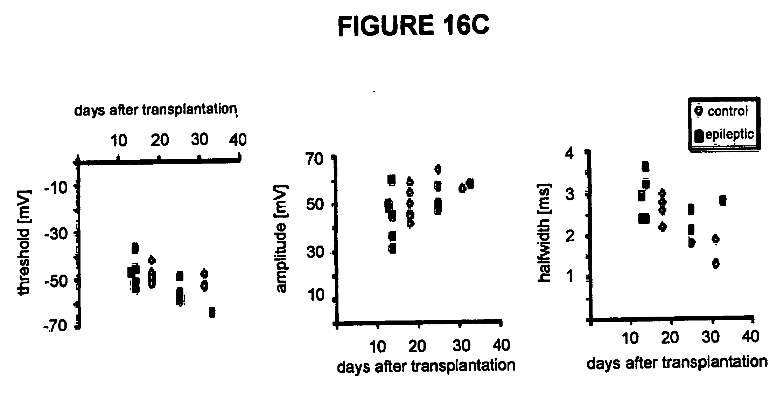

Figure 16 shows the discharge behavior of ESNs. A1: EGFP backdiffusion into the recording pipette after attaining the whole-cell configuration (scale bar: 25 µm). A2 - A3: Biocytin staining of recorded neurons with Texas red avidin (A2, scale bar: 50 µm) or permanent DAB staining (A3, scale bar: 50 µm). B: Current-clamp recordings. The upper panel corresponds to recordings from control rats and the lower panel to recordings in pilocarpine-treated rats (upper trace: voltage recording, lower trace: current injection step). B1: Spontaneous pacemaking activity of ESNs. B2: Single action potentials in response to a 3 ms depolarization. B3: ESNs exhibit repetitive discharges in response to a 1 s depolarization and inward rectification after hyperpolarizing current injections. C: Action potential threshold, amplitude and half-width plotted against time after transplantation.

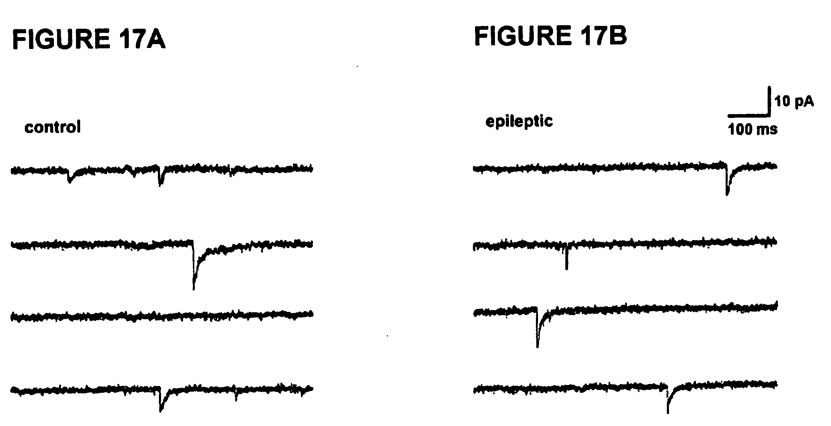

Figure 17 shows spontaneous postsynaptic currents (PSC) in control (A) and epileptic (B) animals.

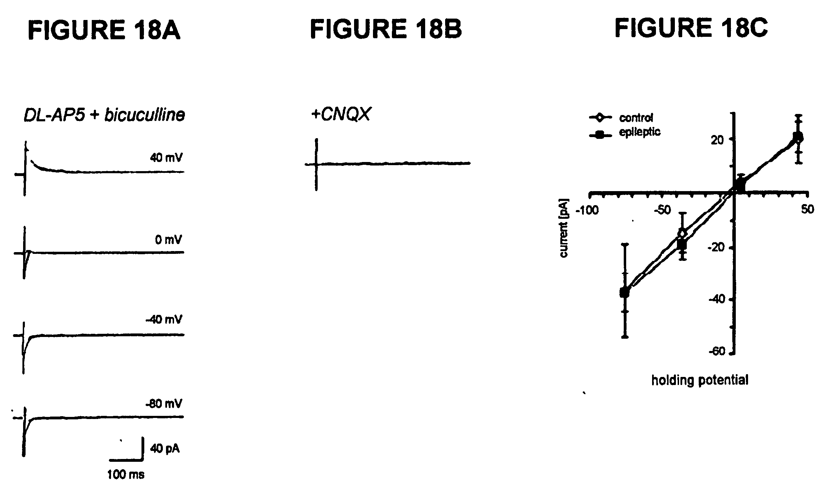

Figure 18 shows excitatory postsynaptic currents. A: Non-NMDA receptor-mediated evoked PSCs at different holding potentials. Averages from 5 to 9 traces are shown. B: PSCs were blocked by CNQX. C: The reversal potential was -2.2 ± 1.4 mV (n = 4) in ESNs of control and 0.5 ± 5.8 mV (n = 6) in ESNs of pilocarpine-treated rats.

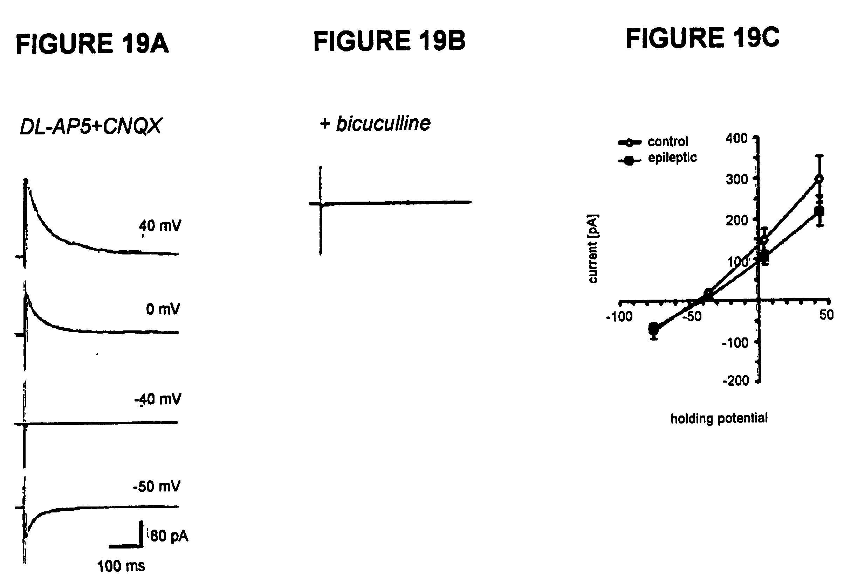

Figure 19 shows inhibitory postsynaptic currents. A: GABAA receptor-mediated evoked PSCs at different holding potentials. Averages from 5 to 9 traces are shown. B: PSCs were blocked by bicuculline. C: The reversal potential was -47.9 ± 2.2 mV (n = 4) and -43.9 ± 1.4 mV (n = 5) in ESNs of control and pilocarpine-treated rats, respectively.

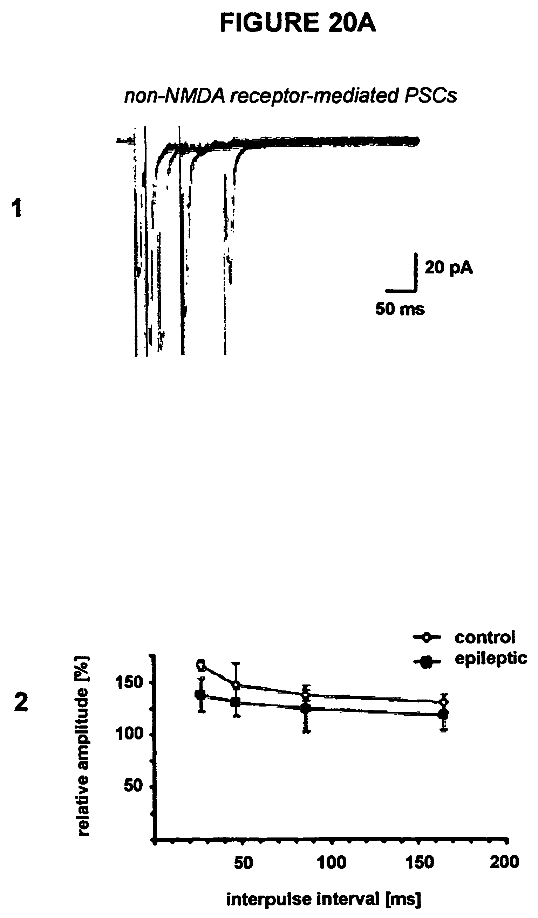

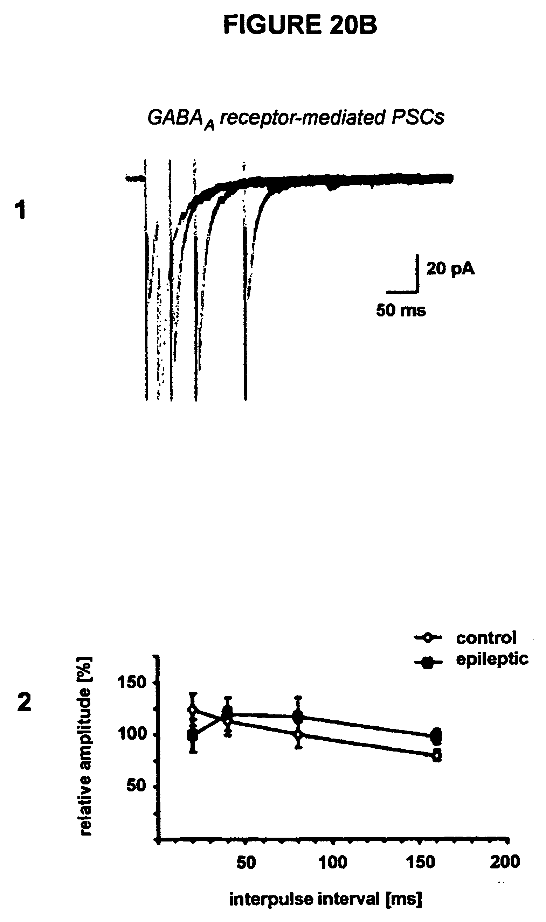

Figure 20 shows paired-pulse plasticity of non-NMDA (A) and GABAA (B) receptor-mediated PSCs. A1, B1: Superimposed averaged PSCs for different interpulse intervals. The leftmost trace is the reference sweep. A2, B2: Ratios of second to first PSC at different interpulse intervals (20, 40, 80 and 160 ms).

### DETAILED DESCRIPTION OF THE INVENTION

The invention can be defined by the appended claims.

### I. Definitions:

As used herein, the term "neural precursor cell" refers to a cell, such as a neural stem cell or a neural progenitor cell, which has become committed to neural cell lineage, but which is not yet terminally differentiated. Thus, the "neural precursor" cell can generate progeny that are either neuronal cells (such as neuronal precursors or mature neurons) or glial cells (such as glial precursors, mature astrocytes, or mature oligodendrocytes). A "neural precursor" cell expresses characteristic markers. Such markers are expressed on the surface or informally by neural precursor cells, and include but are not limited to, polysialyated NCAM, the intermediate filament protein nestin, Vimentin, Musashi-1 and the transcription factor Pax-6. When a neural precursor cell differentiates into mature glia and neurons, nestin is gradually replaced by cell type-specific markers such as GFAP (for astrocytes P or neurofilament (for neurons). Thus, by assessing a combination of neural precursor markers such as nestin with other cell type-specific markers, one of ordinary skill in the art is able to stage the individual neural cell differentiation.

As used herein, the term "embryonic stem (ES) cell" refers to an undifferentiated embryonic cell having pluripotency and the ability to self-replicate. Embryonic stem cells can be established from embryonic cells isolated from embryos at the blastocyst-stage of development with the proviso that said ES cells are not derived by the destruction of a human embryo. Embryonic stem cells are well known to a person of ordinary skill in the art. See, e.g., WO 97/37009, entitled "Cultured Inner Cell Mass Cell-Lines Derived from Ungulate Embryos," Stice and Golueke, published Oct. 9, 1997, and Yang and Anderson, 1992, Theriogenology 38: 315-335. Embryonic stem cells may be cultured with or without feeder cells. The embryonic stem cells express on their cell surface certain types of markers characteristic of the embryonic stem cells. A non-limiting example of markers for human embryonic stem cells includes but is not limited to, alkaline phosphatase, Oct4, SSEA-3, SSEA-4, TRA-1-60, TRA-1-81. In addition, a non-limiting example of markers for mouse embryonic stem cells includes but is not limited to, alkaline phosphatase, Oct4 and SSEA-1.

As used herein the term "neural cell" includes both nerve cells (i.e., neurons, e.g., uni-, bi-, or multipolar neurons) and neural cell precursors and glial cells (e.g., macroglia such as oligodendrocytes, Schwann cells, and astrocytes) and glial cell precursors. In a preferred embodiment, the neural cells for use in the invention are mammalian, e.g., human cells, murine cells, porcine cells, bovine cells, etc. obtained from embryonic stem cells.

As used herein, the term "deficient adenosine kinase (ADK) gene" means an adenosine kinase gene that has been modified or disrupted to prevent its intracellular expression and/or the expression of a functional ADK protein. By "functional ADK protein" is meant an ADK protein that reduces the level of adenosine release by the cell expressing that protein. As used herein, the terms "disruption" and "disrupted" refer to a mutation in a region of the ADK gene so as to decrease or inactivate expression of the ADK polypeptide in cells as compared to cells comprising the wild-type sequence of the gene. Furthermore, the mutation results in at least partial or complete loss of adenosine kinase function.

As used herein, the term "defect", when used in reference to the ADK gene, refers to a mutation to either coding or regulating sequence of the ADK gene that reduces the production of functional ADK protein by the gene. The ADK gene used herein refers to a nucleic acid from a mammal necessary and sufficient to produce an ADK protein. ADK protein is an abundant enzyme in mammalian tissues that catalyzes the transfer of the gamma-phosphate from ATP to adenosine, thereby serving as a potentially important regulator of concentrations of both extracellular adenosine and intracellular adenine nucleotides. Adenosine has widespread effects on the cardiovascular, nervous, respiratory, and immune systems and inhibitors of ADK could play an important pharmacological role in increasing intravascular adenosine concentrations and acting as anti-inflammatory agents. For example, a human ADK protein consists of 345 amino acids with a calculated molecular size of 38.7 kD and without any sequence similarities to other well-characterized mammalian nucleoside kinases. In contrast, 2 regions were identified with significant sequence identity to microbial ribokinase and fructokinases and a bacterial inosine/guanosine kinase. Thus, ADK is a structurally distinct mammalian nucleoside kinase that appears to be akin to sugar kinases of microbial origin.

As used herein, the term "deficient in the production of adenosine kinase" means that due to a mutation on the adenosine kinase gene, the production of adenosine kinase polypeptide is reduced in a cell containing the mutant adenosine kinase gene relative to the production of adenosine kinase polypeptide in a cell containing the wild-type adenosine kinase gene. Preferably, the production of adenosine kinase polypeptide is reduced by at least about 25%, or about 35%, or 50%, or about 65%, or 80%, or 90%, or greater. More preferably, the mutation on the adenosine kinase gene results in complete inactivation, so that there is no production of adenosine kinase polypeptide.

As used herein, the term "an increased level of adenosine" refers to the amount of adenosine released from a genetically modified cell that is higher than that of a corresponding wild-type cell. Preferably, the genetic modification is inactivation of the adenosine kinase gene by gene disruption. Preferably, the increase is at least 15%, preferably at least about 25%, or about 35%, or about 50%, or about 75%, or about 90% or greater, including 100% (or two fold), five fold, ten fold, twenty fold, fifty fold or more, relative to the amount of adenosine released by a corresponding wild-type cell.

As used herein, a "therapeutic amount" is an amount of a therapeutic agent sufficient to achieve the intended purpose. For example, a therapeutic amount of adenosine is an amount sufficient to reduce epileptic activity at a target area. As used herein, "epileptic activity" is defined as any activity that increases the probability of spontaneous or induced generation of synthronous discharges. Typical features of epileptic activity are mainly behavioral seizures such as convulsions, but also include absence seizures, auras, and ictal spike and wave discharges as well as interictal spike activity, which can be identified in electroencephalograms. Also as used herein, a "reduction in epileptic activity" refers to a decrease in any of the above described features, preferably in quantitative terms by about 10%, about 15%, about 25%, about 30%, about 50%, about 60%, about 70%, about 75%, about 85%, about 95%, or greater. The therapeutic amount of a given therapeutic agent will vary with factors such as the nature of the agent, the route of administration, the size and species of the animal to receive the therapeutic agent, and the purpose of the administration. The therapeutic amount in each individual case can be determined empirically without undue experimentation by a skilled artisan according to established methods in the art.

As used herein, the term "polymer suitable for implantation in a mammal" means a natural or synthetic polymer that permits passage of nutrients and materials necessary for maintenance of encapsulated cells, permits escape of adenosine released by the encapsulated cells, and does not provoke an immune or inflammatory response to the polymer in the mammal into what it is implanted.

As used herein, the term "subject" refers to any human or non-human organism.

As used herein, "a neuron" is a type of cell that carries signals between the brain and the rest of the body or within the brain. Each neuron has a cell body, an axon, and dendrites. The tip of an axon is the growth cone and is responsible for navigation. Neurons can make multiple contacts with one or more neurons. The organization of the contacts determines the overall function of the nervous system. The axons are surrounded by an insulating layer or myelin sheath formed by the Schwann cells (Tortora, 1992) or by oligodendrocytes.

As used herein, the term "glial cells" refers to all types of central nerve system (CNS) cells that cannot receive or transmit nerve signals. Generally, "glial cells" include astrocytes, oligodendrocytes, ependymal cells and their precursors. These glial cells perform various activities that can be regarded as performing supporting, housekeeping, and "nursing" functions within the CNS. Neuroglia have high-affinity transmitter uptake systems, voltage-dependent and transmitter-gated ion channels, and can release transmitters. Glia cell-specific markers have been identified. The markers for glia cells include but are not limited to intermediate filament typical for astrocytes (GFAP), calcium-binding protien typical for astrocytes (S100 beta), proteoglycan-component typical for glial precursors (NG2), surface antigen typical for glial precursors (A2B5), RIP for oligodendrotytes, RC2 for radial glia, 04GALC and 01GALC for oligodendrocytes, CNP, PLP, MBP (myelin components geenrated by oligodendrocytes).

As used herein, the term "chronic local dose" refers to an amount of a therapeutic agent administered to a particular tissue site of a subject. The therapeutic agent is not distributed throughout the entire body, thus, the local delivery exhibits therapeutic effects without involving the side effects associated with systemic administration. As used herein, "tissue" in the term "a particular tissue site" refers to any tissue in a biological organism. It may include but is not limited to, lung, heart, blood, liver, muscle, brain, pancreas, skin, central nervous system and others. The term "chronic" means that the release of the therapeutic agent is sustained over a period of at least one day, preferably at least several days, and more preferably up to a week or a month or longer.

### II. Embryonic Stern Cells With Gene Deficiency

One aspect of the present invention is embryonic stem (ES) cells having a deficient ADK gene on both alleles. These genetically engineered ES cells are powerful tools for therapy and cell-based studies. For example, ADK-/- ES cells will release adenosine at a higher level than the corresponding wild-type ADK ES cells, thus, rendering them useful for the *in vivo* treatment of disorders involving insufficient adenosine levels. There are a number of methods to produce ADK deficient ES cells. One method involves gene targeting technology. In general, the gene targeting technology to produce gene deficient ES cells includes two steps. First, a heterozygous ES cell line having a deficient gene on only one ADK allele is produced. Second, homozygous ES cells with the gene deficiency on both alleles are derived from the heterozygous ES cells by disrupting the second allele.

As a reference a homozygous ES cell line can be produced by isolating ES cells containing wild-type ADK gene from mammalian blastocysts with the proviso that said ES cells are not derived by the destruction of a human embryo, disrupting the ADK gene in the ES cells, and either introducing the ES cells containing the disrupted ADK gene into a mammalian embryo to generate first an animal with a heterozygous disruption (one allele inactivated) and subsequently an animal with a homozygous disruption, or subjecting the resulting heterozygously disrupted ES cell line to a second round of gene targeting to disrupt the second allele of the same gene thus generating a cell line homozygous for the gene disruption.

### Isolation of Mouse and Human ES cells

The ES cells isolated from the inner cell mass of a preimplantation embryo are termed embryonic stem (ES) cells. Methods of isolating the ES cells are well known to one of ordinary skill in the art. See Evans, MJ and Kaufinan, 1981, Nature, 292:154; Martin, GR, 1981, PNSA USA, 78:7634; and WO9520042. See also US20030104616 for isolating human embryonic stem cells. Said methods are with the proviso that said ES cells are not derived by the destruction of a human embryo.

### Disruption of ADK Gene

The disruption of the ADK gene can be accomplished by way of insertion, deletion, or replacement of a nucleic acid sequence in a region of the native ADK gene. In one example, the ADK gene is disrupted by gene knockout technology, which includes disrupting the ADK gene in germline cells and producing offspring from these cells. See Huber, A. et al., "Grafts of adenosine-releasing cells suppress seizures in kindling epilepsy," PNAS USA,98(13):7611-7616 (June 19,2001); and Boison, D., et al, "Neonatal hepatic steatosis by disruption of the adenosine kinase gene," PNSA USA, 99(10):6985-6990 (May 14, 2002).

In one embodiment, disruption of the ADK gene in the ES cells involves a specific gene-targeting vector, which is transfected into the ES cells. The vector contains a replacement construct, with two regions of homology to the ADK target gene located on either side of a positive selectable marker. The selectable marker sequence interrupts the coding region for the ADK gene, such that homologous recombination results in gene knock-out. The transfection of the vector can be accomplished in a variety of ways well known in the art, which include for example, electroporation, microinjection, lipofection, and calcium phosphate treatment. When the target vector is introduced into the ES cells, a double crossover event will replace the target gene sequences irreversibly with the replacement construct sequences. The replacement sequences on the vector can result in a deletion and/or point mutations in the gene, as well as an insertion of exogeneous sequences into the gene (a gene disruption). Correct clones are selected by detecting the presence of the positive marker. Preferably, the positive marker is an antibiotic resistance gene. Alternatively, the positive marker is a gene, such as beta-galactosidase or GFP, whose expression or presence in the genome can easily be detected. See Babinet C. and Cohen-Tannoudji M., 2001, An Acad Bras Cienc. Sep;73(3):365-83.

In another embodiment, disruption of the ADK gene in the ES cells can be accomplished by site-specific recombination such as the Cre-loxP recombination system. Site-specific recombination provides an efficient, complementary set of methods for manipulating the target gene, and is in this instance mediated by an exogenously supplied recombinase. For example, one recombinase used is Cre, which is isolated from bacteriophage P1. Cre catalyzes a recombination between two 34bp sequences referred to as loxP. When Cre is expressed, any DNA between two loxP sites will be excised. To accomplish the ADK gene disruption, two mammalian strains are needed. The first strain expresses Cre, and the second strain has the loxP sites flanking the ADK gene sequence to be deleted. By crossing these two strains, the ADK gene sequence will be deleted only in the tissue expressing Cre. See Liu, JL, et al., 2000, Proc Soc Exp Biol Med., 223(4):344-51. This permits the development of animals with tissue-specific knockout of ADK gene function. Alternatively, Cre protein can be introduced by viral gene transfer, protein transduction and other methods well known in the art. For disruption of the ADK gene in human ES cells, see Zwaka and Thomson, "Homologous recombination in human embryonic stem cells," Nat. Biotech., 21(3): 319-321 (2003).

In one embodiment, human ES cells can be disrupted by homologous recombination with a specific gene-targeting vector. For example, the targeting vector contains a replacement construct, with two regions of homology to the ADK target gene located on either side of a positive selectable marker. The selectable marker interrupts the coding region for the ADK gene, such that homologous recombination results in gene knock-out. For example, the targeting vector can be constructed by insertion of two reporter genes cassettes into the ADK gene: IRES-EGFP containing an internal ribosomal entry site (IRES) of the encephalomyocarditis virus and the EGFP gene encoding the enhanced green fluorescence protein (EGFP), and IRES-neo containing an internal ribosomal entry site (IRES) of the encephalomyocarditis virus and the gene neo encoding neomycin resistance. The two reporter gene cassettes are flanked by two homologous arms. The described linearized targeting vector is then introduced into human ES cells and homologous recombination is selected by assaying G418 resistance. In order to obtain human ES cells containing a deficient ADK gene on both alleles, heterozygous human ES cells with a deficient ADK gene on one allele can go through a second round of homologous recombination with the same targeting vector. Human ES cells containing a deficient ADK gene on both alleles can be identified by conventional methods well known in the art such as PCR, or Southern blot, or Northern blot, or Western blot, or positive selective markers, all as discussed above.

Alternatively, chemical mutagenesis can be employed to introduce mutations into the ADK gene in the ES cells and to generate adenosine-releasing ES cells. The chemical mutagenesis involves use of a mutagenic agent in the ES cells. The mutagenic agents, such as N-ethyl-N-nitrosourea (ENU) or ethylmethanesulphonate (EMS), normally result in point mutations in DNA, leading to a variety of genetic lesions that are expressed as complete loss of function, partial loss of function, or gain of function of alleles. For example, ENU is used primarily as an *in vivo* mutagen of the male germline. More recently, ENU and EMS have been used to effectively mutagenize mouse embryonic stem (ES) cells. Mutations in DNA may be measured by detecting resistance to chemical agents, or phenotype changes or gene function changes, etc. See O'Brien, TP, et al., J. Physiol., 554(1):13-21 (2003); Huber, A. et al., 2001, PNAS USA, 98(13): 7611-7616.

### Animal Model for Maintaining ADK-ES Cells in Undifferentiated State

After suitable ES cells containing the ADK disruption construct in the proper location have been identified, the ES cells can be maintained in an undifferentiated state and be used to target a second round of knockout to generate a homozygous disruption, or, alternatively can be used to generate a line of knock-out animals. In this aspect, the cells are inserted into an embryo of a mammal. Insertion can be accomplished by microinjection. For microinjection, about 10-30 cells are collected into a micropipet and injected into embryos that are at the proper stage of development to integrate the ES cell into the developing embryo. The suitable stage of development for microinjection into the embryo varies with species, however for mice it is about 3.5 days. The embryos for microinjection are obtained by perfusing the uterus of pregnant females. Suitable methods for accomplishing this are known to one skilled in the art. See Wood, SA. et al., 1993, Nature, 365:87-89, Stewart, CL., in Wassarman, P.M. and Depamphilis, ML. (eds), 1993, Methods Enzymol., Academic Press, NY. pp. 823-855.

While any embryo of the right age/stage of development is suitable for use, preferred embryos are male and have genes coding for a coat color that is different from the coat color encoded by the ES cell genes. In this way, the offspring can be screened easily for the presence of the ADK knockout construct by looking for mosaic coat color (indicating that the ES cell was incorporated into the developing embryo). Thus, for example, if the ES cell line carries the genes for white fur, the ADK knockout embryo will carry genes for black or brown fur.

After the ES cell has been introduced into the embryo, the embryo is implanted into the uterus of a pseudopregnant foster mother. While any foster mother can be used, they are typically selected for their ability to breed and reproduce well, and for their ability to care for their young. Such foster mothers are typically prepared by mating with vasectomized males of the same species. The stage of the pseudopregnant foster mother is important for successful implantation, and it is species dependent. For mice, this stage is about 2-3 days pseudopregnant.

Offspring that are born to the foster mother can be screened initially for mosaic coat color where the coat color selection strategy (as described above) has been employed. In addition, or as an alternative, DNA from tail tissue of the offspring can be screened for the presence of the ADK knockout construct using Southern blots and/or PCR. Offspring that appear to be mosaics are then crossed to each other if they are believed to carry the knockout construct in their germline to generate homozygous ADK knockout animals. If it is unclear whether the offspring will have germ line transmission, they can be crossed with a parental or other strain and the offspring screened for heterozygosity. The heterozygotes are identified by Southern blots and/or PCR amplification of the DNA.

Other means of identifying and characterizing the ADK knockout offspring are available. For example, Northern blots can be used to probe the mRNA for the presence or absence of transcripts encoding either the knocked out ADK gene, the marker gene, or both. In addition, western blots can be used to assess the level of expression of the knocked out ADK gene in various tissues of these offspring by probing the Western blot with an antibody against the protein, or an antibody against the marker gene product, where this gene is expressed. Finally, in situ analysis (such as fixing the cells and labeling with antibody) and/or FACS (fluorescence activated cell sorting) analysis of various cells from the offspring can be conducted using suitable antibodies to look for the presence or absence of the ADK knockout construct gene product.

The heterozygotes can then be crossed with each other to generate homozygous knockout offspring. Homozygotes can be identified by Southern blotting of equivalent amounts of genomic DNA from a mammal that is the product of this cross, as well as a mammal that is a known heterozygote. Homozygous ADK knockout ES cells may be isolated from the homozygotes containing the gene knocked out on both alleles.

Alternatively, ES cells containing the gene knocked out on both alleles may be directly produced from a heterozygous ES cell with the gene knocked out on one allele. The heterozygous ES cells with the gene knocked out in one allele are subjected to a second round of gene disruption. The methods of gene disruption can be any one discussed above, i.e., disruption via homologous recombination, or chemical mutagenesis. Preferably, homologous recombination is used. Homozygous ADK knockout ES cells with the gene knocked out on both alleles can be identified by PCR, or Southern blot, or Northern blot, or Western blot, or positive selective markers, all as discussed above. For example, to obtain mouse ES cells containing a deficient ADK gene on both alleles, heterozygous mouse ES cells with a deficient ADK gene on one allele are isolated from a heterologous ADK knockout mouse. The same replacement vector used to generate the heterozygous ADK knockout mouse is then transfected into the heterozygous ADK ES cells to initiate a second round of the ADK gene disruption. The resulting ES cell clones with ADK knocked out on both alleles are identified by detecting the cell resistance to 6-methylmercaptopurine riboside (MMPR). Cells from the MMPR-resistant clones are analyzed for ADK deficiency on both alleles (ADK-/- ES cells) by methods including PCR, Southern blot, and Western blot etc.

### III. Differentiation of ADK Deficient ES Cells to Neural Lineage

ES cells have the powerful advantage that they can be directed into multiple differentiation pathways *in vitro and in vivo.* Transplantation therapies with embryonic stem cells have focused on directed differentiation and cell replacement in several therapeutic settings such as replacement of dopaminergic neurons in Parkinson's disease, remyelination, spinal cord repair, replacement of insulin-secreting cells in diabetes, or replacement of damaged cardiomyocytes after heart attack (Bjorklund et al., 2002; Brüstle et al., 1999; Klug et al., 1996; McDonald et al., 1999; Soria et al., 2000). Therefore, another aspect of the present invention is directed to neural cells differentiated from the homozygous ES cells with a deficient ADK gene on both alleles. The differentiated neural cells can be neural precursor cells or mature neural cells such as glial cells, all with the deficient ADK gene on both alleles.

The ES cells can be differentiated *in vitro* to yield neural progenitor/precursor cells, neuron or glial cells as well as extra-embryonic cells, such differentiation being characterized by novel gene expression characteristic of specific lineages (markers) as demonstrated by immunocytochemical or RNA analysis. For example, markers specific for glial cells include but are not limited to intermediate filament typical for astrocytes (GFAP), calcium-binding protien typical for astrocytes (S100 beta), proteoglycan-component typical for glial precursors (NG2), surface antigen typical for glial precursors (A2B5), RIP for oligodendrotytes, RC2 for radial glia, 04GALC and 01GALC for oligodendrocytes, CNP, PLP, MBP (myelin components geenrated by oligodendrocytes). Markers specific for neuronal lineage include but are not limited to beta-III tubulin, MAP2, NeuN, neurofilament, synaptophysin and other synaptic proteins etc. The establishment of neural progenitor/precursor cells from embryonic stem cells and more preferably a pure preparation of neural progenitor/precursor cells and even more preferably a neural progenitor/precursor cell line can be achieved by any one or a combination of the following approaches.

In one preferred embodiment, the method involves transfer of undifferentiated stem cells into culture conditions that on one hand direct differentiation toward a desired somatic lineage, which is the neural lineage in this case, while on the other hand are selective and therefore limit both the differentiation toward unwanted lineages (such as extra-embryonic lineages or endoderm) as well as the survival of differentiated cells from these lineages. See US20020068045. Such culture conditions include transfer into serum free medium supplemented with fibroblast growth factors such as basic fibroblast growth factor (bFGF) and epidermal growth factor (EGF). Any standard serum free medium can be used. Preferably NS-A (Euroclone) or DMEM/F12 (Gibco) is used. More preferably NS-A or DMEM/F 12 supplemented with N2 or B27 (Gibco) is used. Most preferably DMEM/F12 supplemented with B27 is used. In the presence of an appropriate supplement of growth factors such as EGF and basic FGF, the neural progenitors can be cultivated and expanded to establish a cell line. The growth factors inhibit further differentiation of the progenitor cells and promote their proliferation. Culture in the serum free medium and growth factors is selective and therefore limits prolonged proliferation of other types of differentiated cells such as the progeny of the extra-embryonic lineage or definitive endoderm that may coexist in the culture. Therefore the cultivation in these selective conditions can be used to establish an enriched cell line of neural progenitors/precursors. The serum free medium also promotes differentiation towards the neuroectodermal lineage (and possibly other non-neural lineages such as mesoderm). The serum free medium can limit the growth and survival of unwanted cells such as those from the extra-embryonic or endodermal lineages.

To make glial precursor cells and glial cells, ES cells are aggregated to embryoid bodies and plated in a defined medium (See Reference Example 3) that favors the survival of ES cell-derived neural precursors. Cells are then passaged and sequentially propagated through media containing bFGF and EGF, and bFGF and PDGF. These conditions yield an isomorphous population of round to bipolar cells with immunoreactivity to the monoclonal antibody A2B5, which recognizes a membrane epitope typically expressed in glial precursors. Upon growth factor withdrawal, the cells differentiate into oligodendrocytes and astrocytes. See Brustle, O., et al., 1999, Science, 285:754-756; Brustle, O., et al., 1997, PNAS USA, 94:14809-14814.

The neural progenitor/precursor cells can be cultivated as spheres or as a monolayer. Subculturing can be conducted mechanically. Scraping is preferred to propagate monolayer cultures. However, any mechanical method such as trituration or cutting can be used to subculture the spheres. Most preferably the spheres are sliced into smaller clumps. The progenitors can be expanded to produce a large number of cells. In particular, multipotent neural precursor cells (i.e., precursors which can give rise to neurons, astrocytes and oligodendrocytes) are expanded in the presence of FGF2 (bFGF, 10-20ng/ml). ES cell-derived precursors can also be expanded in the presence of FGF2/EFG and/or FGF2/PDGF as described in Brustle, O., et al., 1999, Science, 285:754-756 and Bogler et al., Proc. Natl. Acad. Sci. USA, 87:6368-6372 (1990).

The neural progenitor/precursor cells that are generated directly from undifferentiated stem cells have similar properties to the neural progenitors that are generated from differentiating stem cell colonies. They express the same markers of primitive neuroectoderm and neural progenitor cells, such as polysialyated NCAM, the intermediate filament protein nestin, Vimentin and the transcription factor Pax-6. They do not express the transcription factor oct-4. They have a similar growth potential to the neural progenitor/precursor cells derived from differentiating stem cells. They generate differentiated neural cells with similar morphology and marker expression after plating on appropriate substrate and withdrawal of growth factors.

Therefore, the present invention provides an ADK deficient neural progenitor/precursor cell differentiated from an ES cell. Preferably, the neural progenitor/precursor cell is a glial precursor cell. Preferably, the differentiated neural progenitor/precursor cell exhibits increased adenosine release.

The neural progenitor/precursor cells can proliferate *in vitro* for prolonged periods in an undifferentiated neural progenitor state to produce a large number of cells. The neural progenitor/precursor cells can differentiate, under differentiating conditions (as described above) to mature neurons and glial cells. Therefore, the present invention also provides a glial cell differentiated from a neural progenitor/precursor cell. Preferably, the glial cell is an astrocyte or an oligodendrocyte. More preferably, the glial cell has a deficient gene on both alleles. Most preferably, the glial cell has a deficient ADK gene on both alleles and releases a therapeutic amount of adenosine both *in vitro* and in a host subject.

In one embodiment of the invention, *Adk*^{-/-} ES cells are propagated in ITSFn medium and subsequently in FGF2 to obtain pan-neural precursors (see, e.g., Okabe et al., 1996). Pan-neural precursor cells can be further differentiated into neuronal or glial precursors using the differentiation protocol described above. Thus, the present invention contemplates that adenosine release may be obtained from the pan-neural precursor cells in addition to the neural precursors and glial precursors described above. Moreover, the adenosine releasing pan-neural precursor cells are capable of differentiating into neuronal precursors and neurons, both *in vitro* and *in vivo.* Thus, in one embodiment, *Adk*^{-/-} ES cell-derived pan-neural precursor cells are implanted into the brin of an epileptic animal. The implanted pan-neural precursor cells integrate into the brain, differentiate into neuronal and glial cells, wherein the neuronal cells establish processes and synaptic connections, and demonstrate at least short-term plasticity. Accordingly, the invention relates to a method for the treatment of epilepsy by implanting in the brain of an individual in need of epilepsy treatment, a population of *Adk*^{-/-} ES-cell derived neural precursor cells (e.g., pan-neural precursor cells), which are able to integrate into the brain and differentiate into adenosine producing neurons and glia.

### IV. Dosage and Mode of Administration

Adenosine acts as a neuromodulator to inhibit neuronal firing and the release of neurotransmitters, as an inhibitor of platelet aggregation, as a cardiac depressant, and as a vasodilator, a vasoconstrictor, as in the renal afferent arterioles and in the skin, as an immunosuppressant, and in a variety of other systems. As described above, adenosine is useful for the treatment of epilepsy. Adenosine also has been demonstrated to exhibit potent analgesic actions in chronic and neuropathic pain. See Jarvis et al., 2002, Pharmacol Biochem Behav, 73: 573-581; Kowaluk and Jarvis, 2000, Opin. Investig Drugs, 9:551-564; Sawynok, et al., 1998, Pain, 74:75-81. Adenosine also has been described as a mediator promoting oligodendrocyte differentiation and myelination. See Stevens, B. et al., 2002, Neuron, 36:855-868. Adenosine also offers a unique mechanism of ischemia therapy. See Sommerchild and Kirkeboen, 2000, Acta Anaestthesiol Scand, 44:1038-1055.

Accordingly, another aspect of the present invention provides methods of chronic local delivery of the genetically engineered neural cells to a tissue site of a subject associated with neuronal activity. Preferably, the neuronal activity is epileptic activity. Means of delivery include but are not limited to, implantation of cells encapsulated in a suitable polymer and direct implantation of cells.

One method is to encapsulate genetically engineered cells into a semipermeable polymer membrane and to transplant the polymer membrane into a tissue site of a host subject. Such method may achieve local, long-term chronic delivery of a therapeutic substance with the capability of regulating release of the substance. See U.S. Pat. No. 5,573,528 for description of encapsulation of compounds and cells. In one embodiment, ADK-/- cells are encapsulated within a polymer membrane. Preferably, the ADK-/- cells are neural precursor cells. More preferably, the ADK-/- cells are glial cells. The encapsulated polymer membrane is then transplanted into a tissue site of a host subject for releasing adenosine. Preferably, the tissue site is central nervous system, such as brain.

The semipermeable polymer membrane allows molecules and agents secreted from the cells within the membrane to enter into the environment. The polymer can be synthetic or natural. Examples of polymer that can be used for this purpose include polyethersulfone (PES), polyacrylonitrile-co-vinyl chloride (P[AN/VC], poly(lactic acid), poly(lactic-co-glycolic acid), methylcellulose, hyaluronic acid, collagen, and the like. Delivery of encapsulated cells within a polymer membrane can avoid host rejection and immune response to cells, and problems associated with rejection and inflammation. In addition, cells contained within the polymer membrane are shielded by the wall of the polymer (i.e., the walls of the individual fibers, fibrils, films, sprays, droplets, particles, etc.) from immune surveillance while still maintaining cell viability and allowing transport of molecules, nutrients and metabolic products through the polymer walls. The grafting of polymer-encapsulated cells has been developed by Aebischer et al., 1991, Transplant, 111:269-275, and has been successfully used with both non-human primates and humans (Aebischer et al., 1994, Transplant, 58:1275-1277). See also U.S. Pat. No. 6,110,902.

In one example, cells are encapsulated by first embedding them into a matrix of either collagen, agarose or PVA (polyvinylalcohol). Subsequently, the embedded cells are injected into hollow fibers made of polypropylene of a 60:40 copolymer of polyacrylnitrile:polyvinylchloride. The fibers are cut into pieces and end-sealed for implantation. Preferably, the cells encapsulated are adenosine-releasing cells. More preferably, the cells are ADK-/- cells that release an increased amount of adenosine relative to the corresponding wild-type cells. Most preferably, the ADK-/- cells are neural cells including neural progenitor/precursor cells, or mature neural cells such as neuronal cells and glial cells. Furthermore, the encapsulated cells preferably have about 20,000 to about 2,000,000 neural cells.

Cells used for encapsulation can be of exogenous origin. By the term "exogenous" is meant cells obtained from sources other than the subject in which they are implanted for treatment. Exogenous cells can be from other organisms of the same species (such as human-derived cells for use in a human patient). Exogenous cells can also be from heterologous sources, i.e., from a species distinct from the subject to be therapeutically treated (such as mouse cells for use in a human). When xenogeneic cells are used for transplantation, encapsulation of the cells into semipermeable polymer membranes provides a number of advantages. For example, the cells are immunologically isolated and therefore have an extended in vivo viability. Additionally, encapsulated cells can be more easily removed from a subject, if necessary.

Apart from exogenous sources, neural precurror cells can also be taken from an isogenic source, i.e., from the subject who is to receive the encapsulated cells. After harvesting the cells from the subject, the cells can be genetically modified, then reimplanted back to the subject. Since the cells are isogeneic, no immune response is to be expected. Therefore encapsulation of such cells may not be required.

In one aspect, the adenosine-releasing cells are immortalized. For example and not by way of limitation, cells can be conditionally immortalized. The tsA 58-system described herein below is one method of conditionally immortalizing cells providing the added advantage that the cells grow well in tissue culture at reduced temperatures, yet discontinue division once implanted into a patient and maintained at 37°C. In addition to conditional immortalization, adenosine-releasing cells can be constitutively immortalized by methods well known in the art. Examples of constitutive immortalization methods are transfection with constructs expressing large T antigen, or immortalization by Epstein Barr virus.

Alternatively, another method of delivering cells into a host subject is to directly transplant the cells into the target area of a tissue site. Once transplanted, these cells survive, migrate and integrate seamlessly into the host tissue. Preferably, the cells transplanted are neural precursor cells. More preferably, the cells transplanted are ADK-/- neural precursor cells that release adenosine at an increased level than the corresponding wild-type neural precursor cells. More preferably the neural precursor cells are differentiated into mature neural cells such as glial cells both *in vitro* and in the host subject. Most preferably, the glial cells are ADK-/- glial cells that release a therapeutic amount of adenosine both *in vitro* and in the host subject.

In one embodiment, the neural precursor cells are directly transplanted into the nervous system of the host subject. Preferably, the neural precursor cells are transplanted into a developing nervous system. When transplanted into a developing nervous system, the neural precursor cells will participate in processes of normal development and will respond to the host's developmental cues. The transplanted neural precursor cells will migrate along established migratory pathways, will spread widely into disseminated areas of the nervous system and will differentiate in a temporally and regionally appropriate manner into progeny from both the neuronal and glial lineages in concert with the host developmental program. The transplanted neural precursor cell is capable of non-disruptive intermingling with the host neural precursor cells as well as differentiated cells. The transplanted cells can replace specific deficient neuronal or glial cell populations, restore defective functions and can express foreign genes in a wide distribution.

Also preferably, the neural precursor cells are transplanted into a developed nervous system. The transplanted neural precursor cells can form a stable graft, migrate within the host nervous system, intermingle and interact with the host neural progenitors and differentiated cells. They can replace specific deficient neuronal or glial cell populations, restore deficient functions and activate regenerative and healing processes in the host's nervous system. More preferably, the transplanted cells can express foreign genes in the host's nervous system. More preferably, the stable graft is a graft established in the central nervous system or the peripheral nervous system.

Another aspect of the present invention provides a method of treating neural disorders associated with neuronal activity by using cell-based delivery approaches discussed above. Preferably, the present invention provides a method of treating epilepsy. Also preferably, the present invention provides a method of ameliorating forms of acute and chronic pain, in particular carcinoma-induced pain, neurogenic pain, postoperative pain, neuropathic pain, and neuralgic pain. Specific pain syndromes that can be treated with the methods and compositions of the invention include pain associated with soft tissue disease and peripheral damage (e.g. acute trauma, osteoarthritis, rheumatoid arthritis, burns, episiotomy), spinal pain, musculo-skeletal pain, upper-extremity pain, myofascial pain syndromes, headache, deep and visceral pain syndromes (e.g. heart pain, muscle pain, eye pain, orofacial pain, abdominal pain, gynecological pain and pain during labour), pain associated with nerve and root damage (e.g., peripheral nerve disorders or infections, amputation-induced pain, peripheral neuropathies, tic douloureux and atypical facial pain, arachnoiditis), carcinoma-induced pain (particularly that involving bone and soft tissue carcinoma and metastases), and central nervous system-induced pain (such as central pain due to spinal cord or brain stem damage). Many patients with chronic pain or carcinoma pain do not respond to opiates. The present invention provides another means for treatment of these patients.

In order to supply a local chronic dose of adenosine to treat epilepsies, adenosine-releasing cells or polymers are stereotactically implanted into or near the epileptogenic focus. For treatment of chronic pain syndromes, implantation of adenosine releasing polymers, cells or encapsulated cells is done intrathecally or epidurally to block pain transmission in the spinal cord. Alternatively, adenosine releasing cells or encapsulated cells can be implanted into a peripheral nerve plexus, e.g., plexus axillaris or plexus femoralis that is involved in pain transmission in the pain syndrome. For all indications, dosages can be altered by, for example, varying the size of the implant, using different cell numbers or compound concentrations in the implant, and by using different polymers.

The therapeutic dose and regimen most appropriate for patient treatment will vary with diseases or conditions to be treated, and according to the patient's weight and other parameters. U.S. Pat. No. 6,110,902 has demonstrated that a dose of adenosine equivalent to about 250 ng/adenosine/day administered constantly over several days up to two weeks can ameliorate epileptic symptoms. It is expected that much smaller doses, e.g., in the 25 ng/adenosine/day range, and longer duration of treatment, will also produce therapeutically useful results, i.e., a statistically significant decrease in epileptic events or severity. The dosage sizes and dosing regimen most appropriate for human use are guided by the results herein presented, and can be confirmed in properly designed clinical trials.

An effective dosage and treatment protocol can be determined by conventional means, starting with a low dose in laboratory animals and then increasing the dosage while monitoring the effects, and systematically varying the dosage regimen. Numerous factors can be taken into consideration by a clinician when determining an optimal dosage for a given subject. Primary among these is the amount of adenosine normally produced by nerve cells, which is on the order of 0.03 to 0.3 µM (extracellular concentration) in the resting state, rising to 30 to 300 µM after nerve cell stimulation (Corradetti et al., 1984, Eur. J. Pharmacol. 104:19-26; Fredholm, 1995, NIPS, 10:122-128; and Aulrich and Huguenard, 1995, Neuron 15:909-918). Additional factors include the size of the patient, the age of the patient, the general condition of the patient, the particular disease being treated, the severity of the disease, the presence of other drugs in the patient, the in vivo activity of the adenosine agonist, and the like. The trial dosages would be chosen after consideration of the results of animal studies and the clinical literature. It will be appreciated by the person of ordinary skill in the art that information such as binding constants and Ki derived from in vitro adenosine receptor binding competition assays may also be used in calculating dosages.

A typical human dose of the compound adenosine released locally over an extended period would be from about 1 to 500 ng released per day, preferably from about 5 to 100 ng released per day, and most preferably about 10 to 50 ng released per day.

Accordingly, adenosine-releasing cells or polymers are administered to subjects so as to reduce or ameliorate symptoms associated with epilepsy or chronic pain. Therapeutic endpoints for the treatment of epilepsy include a reduction of disease parameters such as seizure frequency, seizure severity and EEG-abnormalities. Preferably, the reduction is at least about 10%, or about 15%, or about 25%, or about 40%, or about 50%, or greater. Effectiveness of pain syndrome treatment is assessed by (1) an improvement, as measured by subjective pain rating scales, in pain severity, pain quality and pain threshold; and/or (2) normalization or reduction of pain-induced increases in blood hormone levels. In one example, the antieplieptic effectiveness of the delivered adenosine-releasing cells is measured in a kindled animal. See Boison, D. et al, 2002, Epilepsia, 43(8):788-796; Huber, A. et al., 2001, PNAS USA, 98(13):7611-7616. In this approach, four days after grafting the adenosine-releasing cells into the lateral brain ventricle of fully kindled rats, i.e. rats, which reproducibly react with a grade 5 seizure after each test stimulus, each rat is given test stimulation either once or three times per week. During a period of four weeks, the behavioral seizure response is scored according to the scale of Racine and averaged over the total number of weekly test stimulation. See Racine, R. et al., 1978, Neurosorg, 3:234-252. Any reduction from grade 5 seizure severity would be considered therapeutically efficient, preferably a reduction to grade 0,1, and 2 seizure activity (lack of convulsions), and most preferably to grade 0 (complete lack of seizures). In addition to a reduction of behavioral seizure activity, the therapeutic effect in treated animals can be determined by a suppression of after-discharge activity in EEG recordings.

For use in inhibiting pain syndromes or epilepsy in a subject, the present invention also provides in one of its aspects a kit or package, in the form of a sterile-filled vial or ampoule, that contains an adenosine-releasing cell. In one embodiment, the package is a sterile-filled vial or ampoule containing an adenosine-releasing cell or cell line. For storage and transport, the adenosine-releasing cell or cell line is preferably frozen. Preferably, the package also contains a cell encapsulation agent, and instructions for its use. Optionally, the package may also contain media and reagents for culturing the adenosine-releasing cell or cell line, and for forming an encapsulated cell implant. In yet another embodiment, the package is a sterile encapsulated adenosine-releasing cell implant.

### EXAMPLES

Examples not pertaining to the invention are for illustrative purposes only.

### Example 1 Genetic disruption of adenosine kinase in mouse embryonic stem cells

The isogenic replacement-type gene targeting vector pAdk- was used to disrupt one allele of the *Adk* gene in the mEMS32 line (Simpson et al., 1997) of embryonic stem cells. One correctly targeted clone was successfully used for the generation of an adenosine kinase knockout mouse (Boison et al., 2002b). The same clone was subsequently used for the genetic disruption of the second allele of *Adk* as follows:

A total of 10⁷ cells heterozygous for the *Adk* knockout were again electroporated with 25 µg of the gene targeting vector pAdk- (Figure 1a). Clones with a genetic disruption of both alleles of *Adk* were selected by using 6-methylmercaptopurine riboside (MMPR, Sigma, Buchs, Switzerland), a prodrug normally activated by ADK to form MMPR-5'-phosphate, which inhibits de novo purine nucleotide biosynthesis (Nord et al., 1996). Fourty hours after electroporation MMPR and guanosine as an enhancer for selection (Sokoloski and Sartorelli, 1987) were added to the standard ES cell culture medium to final concentrations of 25 and 200 µM, respectively. After 4 days of selection a total of 4 colonies had survived. These colonies were isolated and after expansion in standard ES cell medium one of these colonies could be propagated. Cells from this surviving colony were analyzed by PCR and Western Blot analysis and identified as being homozygous for the disruption of the *Adk* gene and as being devoid of any ADK protein.

*Adk* specific PCR: For the analysis of homologous recombination events, DNA was extracted from the targeted ES cells and subjected to PCR with allele-specific primer sets. PCR reactions were performed in a volume of 20µl in 1 x PCR buffer (Invitrogene, Basel, Switzerland) containing µl of genomic DNA, 2mM MgCl₂, 0.5µM of each of three primers, and 200µM of each deoxynucleotide. PCR-primers used were o107, 5'-CTCACTTAAGCTGTATGGAGGTGACCG-3' (sense primer specific for wild-type *Adk,* SEQ ID NO. 1); o108, 5'-AGTCACAGATGCATCTGCAGAGGTGAG-3' (antisense primer specific for wild-type *Adk*, SEQ ID NO. 2); and o109, 5'-ACTGGGTGCTCAGGTAGTGGTTGTCG-3' (antisense primer specific for targeting construct, SEQ ID NO. 3). Amplifications were carried out for 35 cycles at 95°C (15 s), 61°C (20 s), and 70°C (90 s). PCR reactions were performed using the three primers simultaneously. The combination of primers o107 with o108 gave rise to 640 bp products being indicative of a wild-type *Adk* allele, whereas the combination of primers o107 with o109 (Figures 1a and 1b) gave rise to 840 bp products being indicative for the *Adk*-knockout allele.

Western blot analysis: Aqueous protein extracts from embryonic stem cells cultured in the absence of feeder cells were separated on a SDS/10% PAGE gel (30µg total protein loaded) and blotted onto a nitrocellulose membrane according to standard procedures. The blots were probed with a polyclonal rabbit antiserum raised against recombinant mouse ADK (Boison et al., 2002b; Gouder et al., 2004).

A heterozygous embryonic stem cell line with a genetic disruption of one *Adk* allele has previously been generated for the production of an ADK knockout mouse (Boison et al., 2002b). We now used these same cells to subject them to a second "targeting round" with the original *Adk*-specific gene targeting construct pAdk- (Fig. 1a). Since 6-methylmercaptopurine riboside (MMPR) is phosphorylated by adenosine kinase into the toxic metabolite 5'-phospho-MMPR (Nord et al., 1996), after electroporation of 10⁷ heterozygous ADK knockout ES cells with pAdk-, 20 µM MMPR was added to the culture medium for the selection of ADK deficient cells. After 4 days of selection 4 colonies survived, which were subsequently isolated. One of these colonies was propagated further. Cells from this MMPR-resistant clone were analyzed for adenosine kinase deficiency by two independent methods:

To identify the presence of different *Adk* alleles, PCR reactions with a set of the three allele-specific primers o107, o108, and o109 were performed on DNA prepared from wild-type ES cells and the MMPR-resistant clone produced here. The wild-type specific primers o107/o108 gave rise to a 640 bp band indicative of wild-type *Adk,* which became evident in samples taken from wild-type ES cells, while DNA taken from the MMPR-resistant clone resulted in the amplification of an 840 bp knockout-specific product by primers o107/o109 (Fig. 1a,b). These PCR-based results demonstrate that the MMPR-resistant clone contains two disrupted *Adk*-alleles.

To demonstrate the lack of ADK protein in the MMPR-resistant *Adk*^{-/-} clone, a Western Blot analysis was performed with aqueous cell extracts derived from wild-type ES cells (*Adk*^{+/+}) and the MMPR-resistant *Adk*^{-/-} cells by using a polyclonal rabbit antiserum raised against recombinant ADK (Boison et al., 2002b; Gouder et al., 2004). In samples taken from wild-type cells a double band was detected in the size range of 44 to 46 kDa being consistent with two alternatively spliced products (McNally et al., 1997) (Fig. 1c). As expected, no ADK specific protein was detected in samples derived from MMPR-resistant *Adk*^{-/-}-cells (Fig. 1c). These findings demonstrated that both alleles of *Adk* have been disrupted in MMPR-resistant cells, leading to ADK deficiency in these *Adk*^{-/-} cells.

### Example 2. Adenosine release from mouse ES cell-derived glial precursors and glial cells

For the analysis of the amount of adenosine released from ES cell-derived glial cells, single cell suspensions of N3EFL cells were plated at a density of 2.5-4 x 10⁵ cells/cm² onto poly-ornithine coated 6-well tissue culture dishes and cultured at 37°C under 5 % CO₂. For half of the dishes, 24 hours after plating, the medium was changed to N2FP. For glial differentiation of both N3EFL and N2FP cells, the culture dishes were withdrawn from growth factors for a period of four days under the same experimental conditions. For sample collection, the medium was replaced with 1.5 ml of fresh medium that was pre warmed to 37°C and two hours later 200µL of medium was collected and immediately frozen at -20°C for later adenosine analysis. After collecting samples, cells on 2 wells from a replicate plate were trypsinized, counted, and averaged and this count was used for normalization of quantity of adenosine per number of cells. Adenosine was quantified using an enzyme coupled bioluminescence assay of adenosine, which was performed as follows:

Adenosine was metabolized into ATP by using recombinant ADK (own production, Institute of Pharmacology and Toxicology, University of Zurich, Switzerland), recombinant AMP:GTP phosphotransferase (own production, Institute of Pharmacology and Toxicology, University of Zurich, Switzerland), pyruvate kinase (Roche, Mannheim, Germany), GTP, and phosphoenolpyruvate. The formed ATP was then quantified with a luciferase assay (ATP Bioluminescent Assay Kit, Sigma, Buchs, Switzerland). The assay was performed as described elsewhere (Zumsteg and Boison, 2002).

Statistical analysis of the results was done using the unpaired t-test. Adenosine levels from 6 samples were averaged and values from *Adk*^{-/-} cells were compared with the respective *Adk*^{+/+} cells at each stage of differentiation (ES cells, N3EFL and N2FP proliferating cells, and N3EFL and N2FP cells differentiated by growth factor withdrawal). An additional comparison was made between adenosine values from *Adk*^{-/-} cells at the ES stage versus N3EFL proliferating stage, and N3EFL proliferating stage versus N3EFL withdrawn cells; this was also done for the control *Adk*^{+/+} cells.

*Adk*^{-/-} ES cells are a valuable tool for the in vivo therapeutic delivery of adenosine. However, cell transplantation therapies require reliable differentiation of ES cells prior to transplantation, since any residual undifferentiated cells may lead to teratoma formation.

To exclude that ADK deficiency compromises the potential of ES cells to differentiate into glial precursor cells, we compared glial differentiation of *Adk*^{-/-} ES cells with the corresponding wild-type (*Adk*^{+/+}) ES cells in the same glial differentiation protocol. *Adk*^{-/-}and *Adk*^{+/+} glial precursors proliferating in FGF2/EGF (N3EFL) or FGF2/PDGF (N2FP) were labeled with antibodies to A2B5 and nestin. When the percentage of positively *Adk*^{-/-}stained cells was calculated for each antibody and compared to that of *Adk*^{+/+} cells, no significant differences were observed (Table 1; Fig. 2). Following growth factor withdrawal for four days, N3EFL and N2FP cells gave rise to GFAP-positive astrocytes and 04-positive oligodendrocytes (Fig. 2). Both *Adk*^{-/-} and *Adk*^{+/+} cells yielded a preponderance of astrocytic cells with values ranging between 58% and 68%. In contrast, less than 6% of cells from both genotypes reached an 04-positive stage after 4 days growth factor withdrawal (Table 1). Considering the variability between single experiments, no evident deficiency of *Adk*^{-/-} cells in glial differentiation was noted. Thus, Adk deficiency does not affect the differentiation of ES cells into proliferating glial precursors with the potential for astrocytic and oligodendroglial differentiation.

**Table 1. Glial differentiation potential of Adk^{-/-} versus Adk^{+/+} ES cells**

| | **N3EFL** | | | | **N2FP** | | | |
|---|---|---|---|---|---|---|---|---|
| | ***Adk*^{-/-}** | | ***Adk*^{+/+}** | | ***Adk*^{-/-}** | | ***Adk*^{+/+}** | |
| | **PROL** | **DIFF** | **PROL** | **DIFF** | **PROL** | **DIFF** | **PROL** | **DIFF** |
| **A2B5** | 61,0 ±15,4 | nd | 64,8 ±1,9 | nd | 50,7 ±8,2 | nd | 48,9 ±10,6 | nd |
| **Nestin** | 76,4 ±4,70 | nd | 78,4 ±9,0 | nd | 56,3 ±0,9¹ | nd | 61,5 ±15,7 | nd |
| **GFAP** | nd | 67,4 ±8,4 | nd | 65,7 ±12,3 | nd | 68,3 ±7,3 | nd | 58,1 ±0,5¹ |
| **04** | nd | 5,30 ±2,2 | nd | 2,30 ±1,50 | nd | 3,20 ±1,2 | nd | 1,70 ±0,6¹ |

ES cells were subjected to controlled differentiation into FGF2/EGF-dependent (N3EFL) and FGF2/PDGF-dependent (N2FP) glial precursors. Proliferating precursors (PROL) were induced to differentiate by a 4-day growth factor withdrawal (DIFF). Antibodies to A2B5 and the intermediate filament nestin were used to characterize the proliferating precursor cells; differentiated astrocytes and oligodendrocytes were identified using antibodies to GFAP and 04, respectively. Shown are percentages ± SEM of immunolabeled cells, based on 20 high power (40x) fields per coverslip and three ('two) independent experiments. nd = not done.

To determine whether *Adk*^{-/-} ES cell-derived glial cells release adenosine in therapeutic amounts, undifferentiated *Adk*^{-/-} and *Adk*^{+/+} ES cells and proliferating *Adk*^{-/-} and *Adk*^{+/+} FGF2/EGF-dependent (N3EFL) glial precursors were plated in replicate (n=6) on permissive substrates and in appropriate medium. 48 hours later, the medium was changed to fresh medium and samples of medium were collected after incubation for 2 hours. For N2FP samples, N3EFL cells were plated in replicate, and 24 hours later the medium was changed to N2FP medium. 48 hours after this medium change samples were taken as described above. For samples from differentiated glial cells, glial precursors were plated in replicate (n=6), allowed to proliferate for 1-2 days in N3EFL medium or N2FP medium, then growth factors were withdrawn for 6 days. Adenosine levels in the supernatants were determined with an enzyme-coupled bioluminescent assay. As expected, it became evident that *Adk*^{-/-} cells from all three differentiation stages released significantly more adenosine than their *Adk*^{+/+} counterparts (Fig. 3, p<0.0001). It is important to note that adenosine release increased with progressive differentiation of the cells, a finding consistent with a reduced demand for adenosine incorporation into nucleic acid biosynthesis in cells with a lower proliferation rate. In this context, adenosine release in *Adk*^{-/-} cells increased significantly (p<0.0001) from 2.6 ± 0.4 ng adenosine per 10⁵ cells per hour (ES cells) to 11.7 ± 1.7 ng adenosine per 10⁵ cells per hour (undifferentiated N3EFL cells) and 40.1 ± 6.0 ng adenosine per 10⁵ cells per hour (N3EFL cells after growth factor withdrawal). In contrast, corresponding *Adk*^{+/+} cells released 0.17 ± 0.02,0.8 ± 0.1, and 3.1 ± 0.6 ng adenosine per 10⁵ cells per hour, respectively. Undifferentiated FGF2/PDGF-dependent *Adk*^{-/-} glial precursor cells (N2FP) released 2.7 ± 0.8 ng adenosine per 10⁵ cells per hour and fully differentiated N2FP *Adk*^{-/-}glial cells released 11.3 ± 2.7 ng adenosine per 10⁵ cells per hour. Corresponding *Adk*^{+/+} cells released 0.2 ± 0.0 and 1.9 ± 0.7 ng adenosine per 10⁵ cells per hour, respectively.

The present invention relates to a new stem cell based delivery system for adenosine. With the aim of developing an adenosine-based cell therapy for the treatment of pharmacoresistant epilepsy, the adenosine kinase gene was disrupted by homologous recombination in embryonic stem cells (Fig. 1).

ES cells have the powerful advantage that they can be directed into multiple differentiation pathways *in vitro* and *in vivo.* With the genetic disruption of ADK to promote adenosine release, this disclosure provides the first example of engineered ES cells for drug delivery. This accomplishment further broadens the range of applications of ES cell transplantation in therapy.

One prerequisite for therapeutic application is the necessity to guarantee quantitative differentiation of ES cells to avoid tumor formation. It has previously been shown that wild-type ES cells meet this criterion and can be differentiated into pure populations of glial precursor cells (Brüstle et al., 1999). For the therapeutic use *of Adk*-deficient ES cells, however, it must first be demonstrated that the lack of ADK does not compromise the cells' ability to undergo glial differentiation. Indeed, it is herein shown that *Adk*-deficient ES cells maintained their ability to generate astrocytes and oligodendrocytes (Figure 2, Table 1).

In the adenosine release studies herein described, it was demonstrated that, after induction of differentiation into astrocytes and oligodendrocytes, N3EFL and N2FP cells released 40.1 ± 6.0 and 11.3 ± 2.7 ng adenosine per 10⁵ cells per hour, respectively.

The majority of the glial precursors derived from the mEMS32 ES cell line gave rise to astrocytes (Table 1). Considering the high adenosine release levels observed after *in vitro* differentiation, *Adk*^{-/-} astrocytes can provide for transplant-based adenosine delivery to the CNS. Since encapsulated fibroblasts releasing between 8.4 and 19.5 ng adenosine per 10⁵ cells per hour were previously shown to suppress seizure activity when implanted into brain ventricles of hippocampus kindled rats (Huber et al., 2001), adenosine levels released by differentiated *Adk*^{-/-} ES cells are sufficient to provide for seizure suppression.

It is important to note that adenosine release increased with ongoing cellular differentiation. This is likely due to large amounts of purines that are needed for DNA synthesis in rapidly dividing cells, such as undifferentiated ES cells, thereby using up the available pool of purines including adenosine. As cells undergo differentiation and reduce proliferation, less purines are needed and more adenosine is detected in the medium. The increase of adenosine release in differentiated cells further confirms that cellular implants, which are terminally differentiated, can release therapeutic amounts of adenosine.

*Adk*^{-/-} ES derived glial precursor cells as intracerebral implants provided a new strategy for the treatment of pharmacoresistant epilepsy, which continues to remain a major medical problem (Jallon, 1997; Loscher and Potschka, 2002). This is based on two main findings: (i) Pharmacoresistant seizures can be suppressed by the activation of adenosine A₁-receptors; and *(ii)* Ventricular implants of encapsulated ADK deficient fibroblasts suppress seizures in kindled rats (Huber et al., 2001).

Apart from seizure control, *Adk*^{-/-} ES cells can also be used for the treatment of various disorders, such as:
(i) Cerebral hypoxia and ischemia: In these conditions the neuroprotective A₁ receptor mediated properties of adenosine may prevent further tissue damage (Fredholm et al., 2001; Kowaluk and Jarvis, 2000; von Lubitz, 1999).
*(ii)* Chronic and neuropathic pain: Adenosine has been demonstrated to exhibit potent analgesic actions in these conditions (Jarvis et al., 2002; Kowaluk and Jarvis, 2000; Sawynok et al., 1998). Intrathecal implants of ES derived adenosine releasing cells thus provide a means for pain control.
*(iii)* Multiple sclerosis: Adenosine has been described as a mediator promoting oligodendrocyte differentiation and myelination (Stevens et al., 2002). Thus, ES-derived adenosine releasing implants into demyelinated lesions could potentially promote remyelination.
(iv) Myocardial ischemia: The cardioprotective effects of adenosine (Sommerschild and Kirkeboen, 2000) could also be exploited by intracardial implants of *Adk*^{-/-} ES derived cardiomyocytes.

In conclusion, with the generation of *Adk*^{-/-} ES cells and the demonstration of their differentiation potential and adenosine release this disclosure provides useful transplantation therapies covering a wide range of disorders.

### Reference Example 3. Differentiation of ES cells into neural precursor cells

ES cells [line J1 (Li et al., Cell, 69:915-926 (1992)), passage number no large than17] were grown on γ-irradiated embryonic fibroblasts in Dulbecco's modified Eagle's medium (DMEM) containing 20% fetal bovine serum, 0.1 mM 2-mercaptoethanol, nucleosides, nonessential amino acids, and human recombinant leukemia inhibitory factor (LIF, Life Technologies; 1000 units/ml). Cells were passaged once onto gelatin-coated dishes and then aggregated to form embryoid bodies in the absence of LIF. We plated 4-day-old embryoid bodies in tissue culture dishes and propagated them for 5 days in ITSFn medium [DMEM/F12 supplemented with 5 µg/ml insulin, 50 µg/ml transferrin, 30 nM selenium chloride, and 5 µg/ml fibronectin (Okabe et al., Mech. Dev., 59:89 (1996))]. Cells were then trypsinized, plated in polyornithine-coated dishes (15 µg/ml), and propagated in DMEM/F12 supplemented with insulin (25 µg/ml), transferrin (50 µg/ml), progesterone (20 nM), putrescine (100 µM), selenium chloride (30 nM) plus FGF2 (10 ng/ml), and laminin (1 µg/ml). After 5 days, cells were harvested by scraping in calcium- and magnesium-free Hanks' buffered salt solution (CMF-HBSS), triturated to a single-cell suspension, replated at a 1:5 ratio, and grown to subconfluency in the presence of FGF2 (10 ng/ml) and EGF (20 ng/ml). Cells were then passaged at a 1:5 ratio and again grown to subconfluency in the presence of FGF2 (10 ng/ml) and PDGF-AA (10 ng/ml). Human recombinant FGF2, EGF, and PDGF-AA (R&D Systems, Minneapolis, MN) were added daily and the medium was replaced every 2 days. *In vitro* differentiation was induced by growth factor withdrawal.

To initiate differentiation, ES cells were aggregated to embryoid bodies and plated in a defined medium that favors the survival of ES cell-derived neural precursors. Cells were then passaged and sequentially propagated through media containing (i) basic fibroblast growth factor (FGF2), (ii) FGF2 and epidermal growth factor (EGF), and (iii) FGF2 and platelet-derived growth factor (PDGF); the latter is a growth factor combination known to promote the proliferation of glial precursor cells. These conditions yielded an isomorphous population of round to bipolar cells with immunoreactivity to the monoclonal antibody A2B5, which recognizes a membrane epitope typically expressed in glial precursors (Figure 8A). Upon growth factor withdrawal, the cells differentiated into oligodendrocytes and astrocytes. Four days after withdrawal, 38.3 ± 5.8% (mean ± SEM of three experiments) of this population were immunoreactive to 04, an antibody recognizing oligodendrocyte-specific glycolipids. Many of the cells showed a multipolar morphology characteristic for oligodendrocytes (Figure 8B). At the same time, 35.7 ± 6.4% of the cells expressed the astrocytic marker antigen glial fibrillary acidic protein (GFAP) and exhibited a flat morphology typical of cultured astrocytes (Figure 8C). Prolonged growth factor withdrawal for more than 5 days promoted further oligodendroglial differentiation and expression of myelin proteins such as 2',3'-cyclic nucleotide 3'-phosphodiesterase (CNP). Cells growing in FGF2/EGF- and FGF2/PDGF-containing media could be frozen and thawed without losing their potential for oligodendroglial and astrocytic differentiation.

### Example 4 Animal mode for incorporation of ES cell-derived neural precursor cells into the developing brain

Neural precursor cells were trypsinized and triturated to single-cell suspensions in the presence of 0.1% DNase. Timed-pregnant Sprague-Dawley rats were anesthetized with ketamine hydrochloride (80 mg/kg) and xylazine (10 mg/kg), and 0.1-1 × 10⁶ cells were injected into the telencephalic vesicle of each embryo as described.

For transplantation, 4-day embryoid bodies were plated on tissue culture dishes and grown in ITSFn medium. This medium has previously been shown to strongly select for neural precursors. During the first 72 h in ITSFn, a large proportion of the cells died. Most of the remaining cells acquired an elongated phenotype strongly reminiscent of neuroepithelial precursor cells. These cells also expressed nestin, an intermediate filament typically present in neural precursor cells. After 6 days in ITSFn medium, typically more than 80% of the cells were nestin-positive. The remaining cells showed varied morphologies with focal expression of SSEA-1 and keratin 8, i.e., antigens typically expressed in undifferentiated embryonic tissues and primitive ectoderm. After 5-12 days in ITSFn, cells were harvested and used for transplantation. Recipient animals were grafted between embryonic day (E) 16 and E18 and sacrificed between postnatal day (P) 0 and P15. Clusters of donor cells were detected in the ventricles of all successfully injected pups (see below). *In situ* hybridization revealed that large numbers of mouse cells left the ventricle and migrated into various host brain regions, including cortex, striatum, septum, thalamus, hypothalamus, and tectum (Figs. 4 and *6* *A, E, F,* and K and Table 2). The transplanted cells integrated individually into the host tissue and were only detectable by virtue of their genetic difference. The number of incorporated cells varied considerably among individual recipients and brain regions. Quantitative stereology and a detailed assessment of donor cell survival and proliferation will be required to assess what proportion of the transplanted cells integrates into the host brain. Preliminary cell counts have revealed up to 650 incorporated cells in single coronal 50-µm sections.

The differentiation of the incorporated cells was assessed by using antibodies to cell-type-specific antigens in conjunction with a mouse-specific DNA probe or mouse-specific antibodies to M6 and M2. Hybridized neurons expressing the neuronal antigens NeuN and microtubule-associated protein 2 were detected at tel-, di-, and mesencephalic levels (Fig. 5*I* and L). The shape, size, and orientation of these cells were indistinguishable from adjacent host neurons (Fig. 5*I*). Confocal laser microscopy allowed detailed reconstruction of individual phenotypes. ES-cell-derived neurons exhibited characteristic polar morphologieswith segregation of neurites into dendrites and axons (Fig. 5*B-D* and *G*). Both classes of neurites frequently extended several hundred micrometers into the adjacent host neuropil (Fig. 5*G* and *H*). Donor-derived neurons were readily detectable at birth but appeared to undergo further morphological maturation in the postnatal period. The example in Fig. *5M* shows an ES-cell-derived neuron with prominent dendritic spines in the thalamus of a 2-week-old host. Neurons integrating into the host cortex frequently displayed morphologies of projection neurons with long apical dendrites reaching into the superficial cortical layers andbasal axons extending several hundred micrometers into the corpus callosum (Fig. 5*B-D*).² An example of an ES-cell-derived pyramidal neuron in the cortex of a neonatal host is shown in Fig. 5D. The donor neurons generated an extensive axonal network throughout the host gray and white matter, reaching from the most rostral regions such as the olfactory bulb to the brainstem (Fig.6). Within the white matter, donor-derived axons frequently assembled into prominent fiber bundles running alongside host axons through the major axonal trajectories, including the corpus callosum (Fig. 6A), anterior commissure, striatal fiber bundles (Fig. 6*C*), and various other endogenous fiber tracts. Gray matter regions exhibiting dense donor-derived axonal networks included cortex (Fig. 6*A*), hippocampus (Fig. 6*B*), septum, striatum, thalamus (Fig. 6D), hypothalamus, tegmentum, tectum, and brainstem.

Astrocytes generated by the transplanted ES cells were found in a distribution similar to that of donor-derived neurons. The most prominent accumulations were detected in the ventral diencephalon and in tectum. In addition, these cells efficiently incorporated into white matter regions such as the corpus callosum (Fig.4). Donor-derived astrocytes strongly expressed M2, a species-specific antigen frequently used for the identification of mouse astrocytes in xenografts (Fig. 7*A* and *B).* Their astroglial identity was confirmed by double labeling with an antibody to GFAP of cells labeled with either the mouse satellite probe or the M2 antibody (Fig.7*B*). Encountered only occasionally in newborn recipients, these cells were more frequently detected in animals sacrificed at P15 (Table2). ES-cell-derived astrocytes were morphologically indistinguishable from their host counterparts, and GFAP immunofluorescence showed no differences in size or branching pattern between the two populations. Typical for astroglia, donor-derived astrocytes often extended processes to adjacent blood vessels.

In addition to neurons and astrocytes, donor-derived oligodendrocytes were found in the transplanted rat brains. The identity of these cells was confirmed by *in situ* hybridization with the mouse satellite probe and subsequent immunohistochemical detection of CNPase, a marker for myelin and oligodendroglia. ES-cell-derived CNPase-positive oligodendrocytes were only detected in the brains of 2-week survivors and their distribution was restricted to white matter regions such as the corpus callosum and striatal fiber tracts. Size, orientation, and CNPase expression of these cells were indistinguishable from their host counterparts (Fig. 7*C* and *D*).

**Table 2. Differentiation of ES-cell-derived neural precursors after transplanation into the embryonic rat brain**

| | **Parenchyma** | | **Ventricle** | | |
|---|---|---|---|---|---|
| **Age at analysis** | **Neurons** | **Gila** | **NEF** | **AP** | **NNT** |
| PO | TDM | D | + | + | - |
| PO | TDM | T | - | + | - |
| PO | TD | - | + | - | - |
| PO | TM | - | - | + | - |
| PO | TDM | T | - | ++ | - |
| PO | D | D | + | + | - |
| P1 | TDM | - | - | ++ | - |
| P1 | TD | - | - | ++ | - |
| P1 | TDM | - | - | + | - |
| P1 | TD | - | - | + | - |
| P1 | TDM | TDM | - | + | - |
| P1 | TDM | - | - | + | - |
| P1 | TDM | D | - | + | - |
| P1 | TDM | TM | - | ++ | - |
| P15 | DM | DM | + | + | + |
| P15 | TDM | TD | - | - | + |
| P15 | TDM | TDM | + | - | + |
| P15 | TM | TM | + | + | + |
| P15 | T | TD | + | - | + |
| P15 | TDM | TM | + | - | + |
| P15 | TD | TD | + | + | + |

In Table 2, ES cells (line J1) aggregated to embryoid bodies and grown for 5-1 days in ITSFn medium were injected into the ventricle of E16-E18 rats. Recipients were - sacrificed between P0 and P15 and donor-derived neurons were identified by *DNA in situ* hybridization in conjunction with immunohistochemical detection of NeuN or by expression of M6 and unequivocal morphological criteria (presence of dendrites and axons). ES-cell- derived astrocytes were detected with an antibody to M2 or by *DNA in situ* hybridization in conjunction with immunohistochemical detection of GFAP. Intraventricular donor cell clusters were assayed for the presence of nestin-positive neuroepithelial formations (NEF), clusters of undifferentiated, alkaline phosphatase-positive cells (AP; ++, numerous clusters; +, occasional clusters), and differentiated nonneural tissue (NNT). Each row represents one recipient animal. The integration patterns show considerable interindividual variability. There is an increase of glial cells and a decrease of AP-positive cells with increasing survival time, T, telencephalon; D, diencephalon; M, mesencephalon.

### Example 5. Intrauterine transplantation of ES-derived neural precursor cells

Cells were trypsinized and triturated to single-cell suspensions in the presence of 0.1% DNase. Timed-pregnant Sprague-Dawley rats were anesthetized with ketamine hydrochloride (80 mg/kg) and xylazine (10 mg/kg), and 0.1-1 × 10⁶ cells were injected into the telencephalic vesicle of each embryo as described in Brustle, O. et al., 1995, Neuron, 15:1275-1285. See also Brüstle, O., Cunningham, M., Tabar, V., Studer, L. (1997), Experimental transplantation in the embryonic, neonatal, and adult mammalian brain. In: Current Protocols in Neuroscience, Crawley, J., Gerfen, C., McKay, R.D.G., Rogawski, M., Sibley, D., Skolnick, P. (eds.), John Wiley, New York, 3.10.1 3.10.28

### Example 6. Generation of adenosine-releasing fibroblasts by chemical mutagenesis

A baby hamster kidney (BHK) fibroblast monolayer at about 50% confluence was mutagenized during a treatment of 18 hours in complete DMEM containing 200 µg/ml ethyl methanesulfonate and 10% FCS (EMS, Sigma). The cells then were harvested and plated at a density of 1 x 10⁵ cells per 90-mm culture dish. After 3 days, one-third of the cells were replated in complete DMEM containing 10% horse serum and 50 µM adenine 9-β-D-arabinofuranoside (araA; Sigma). In the following, each time the cells reached confluence, one-third of the cells was replated on a new plate and the concentration of araA was increased in steps of 50 µM at each round of replating. When the cells were resistant to 200 µM araA, they were trypsinized and replated at a density of 1 x 10⁵ cells per dish and incubated in DMEM containing 10% horse serum, 10 µM *erythro*-9-[3-(2-hydroxynonyl)]adenine, and 100 µM araA. The adenosine-releasing cell line was isolated and released up to 19 ng of adenosine per 10⁵ cells per hour. It is appreciated by one of ordinary skill in the art that the chemical disruption of the ADK gene was on both alleles, and that both alleles of ADK were mutagenized since cells, which are heterozygous for the disrupted ADK gene (derived from heterozygous ADK knockout mice) do not release adenosine, only cells derived from homozygous ADK knockout mice release significant amounts of adenosine (Zumsteg and Boison, 2002).

### Example 7: Epileptic suppression by paracrine adenosine release

Given the potential benefits of stem cell-derived implants, the present studies evaluated embryonic stem (ES) cell derivatives as a new cellular source for the local therapeutic delivery of adenosine. In a previous study, a stem cell-based delivery system for adenosine was generated by disruption of both alleles of adenosine kinase (ADK) in mouse ES cells (Fedele DE, Koch P, Brüstle O, et al. Engineering embryonic stem cell derived glia foradenosine delivery. Neurosci Lett 2004;370:160-165.). These *Adk*^{-/-} ES cells were differentiated into glial precursor cells and releasedsignificant amounts of adenosine (Fedele et al.). However, it has never been demonstrated that adenosine releasing stem cell-derived brain implants are actually able to suppress seizure activity. Before direct cell implantations can be attempted, where a combination of paracrine and network effects may influence seizure activity, it is essential to safeguard that paracrine effects by adenosine alone are sufficient to prevent seizure activity.

Experiments were performed to study whether ES cells differentiated into embryoid bodies and glial precursors might qualify for this application by determination of the anticonvulsant effect in an established model of temporal lobe epilepsy. To distinguish paracrine effects of *Adk*-/- donor cells from indirect effects due to direct cell transplantation and tissue integration, the cells were encapsulated into semipermeable polymer membranes and implanted into the lateral brain ventricle of kindled rats. In addition, the discrimination of the anticonvulsant action of adenosine, specifically, independent of other factors potentially released from the encapsulated ES cell progeny was accomplished by comparing the results with respective wild-type *Adk*+/+ ES cell-derived implants.

### MATERIAL AND METHODS

### Cell culture

The wild type and genetically altered ES cells (*Adk*+/+ and *Adk*-/-, respectively) used in these experiments were derived from the mEMS32 cell line (Simpson EM, Linder CC, Sargent EE, Davisson MT, Mobraaten LE, Sharp JJ. Genetic variation among 129 substrains and its importance for targeted mutagenesis in mice. Nat Genet 1997;16:19-27.). ES cells were cultured at 37°C under 5% CO2 on a feeder layer of irradiated mouse embryonic fibroblasts in DMEM knockout medium (Life Technologies, Rockville, MD) containing 15% fetal calf serum (FCS, Life Technologies) and supplemented with L-glutamine (200 µM), penicillin (100 U/ml), streptomycin (100 µg/ml), non-essential amino acids (all from Life Technologies), _-mercaptoethanol (Sigma) and leukaemia inhibitor factor (LIF, Chemicon). This medium is referred to as stem cell medium. EBs were generated by first plating *Adk*-/- and *Adk*+/+ ES cells on gelatin-coated dishes without feeders until about 90% confluency. The ES cell cultures were then removed from the plate by incubation with trypsin-EDTA (Life Technologies) at 37°C for 5 min. The ES cells were pelleted, resuspended in ES cell medium without LIF, and cultured for four days in bacteriological dishes (Sterilin, UK).

Glial precursor cells were generated *from Adk*-/- and *Adk*+/+ ES cells using a stepwise differentiation protocol as described (Brüstle O, Jones KN, Learish RD, et al. Embryonic stem cell-derived glial precursors: a source of myelinating transplants. Science 1999;285:754-756; Fedele DE, Koch P, Brüstle O, et al. Engineering embryonic stem cell derived glia for adenosine delivery. Neurosci Lett 2004;370:160-165.). They were routinely cultured on poly-ornithine-coated dishes in N3 medium, which is based on a 1:1 mixture of DMEM with Ham's F12 supplemented with insulin (25 µg/ml), human apo-transferrin (100 µg/ml), progesterone (20 nM), putrescine (100 µM), sodium selenite (30 nM), penicillin (100 U/ml), and streptomycin (100 µg/ml). In addition, laminin (1 µg/ml) was added when plating the cells. To keep the cells in a proliferative state, the growth factors, FGF2 (10 ng/ml) and epidermal growth factor (EGF) (20 ng/ml), were added daily. The N3 medium plus EGF and FGF2 is referred to as N3EFL medium. Upon growth factor withdrawal, these cells differentiate primarily into astrocytes and a smaller fraction into oligodendrocytes (6).

### Cell encapsulation

*Adk-*/*-* and *Adk*+/+ EBs were encapsulated (1.5 x 105 EBs per capsule) into semipermeable polyethersulfone polymer hollow fiber (Akzo Nobel AG, Wupperthal, Germany) membranes (5 mm long, 0.5 mm inner diameter, wall thickness of 50 µm) without a supporting matrix, since EBs form three-dimensional spherical structures in culture. *Adk*-/- and *Adk*+/+ glial precursor cells were encapsulated (3 x 105 cells per capsule) into identical membranes containing a polyornithine-coated polyvinyl alcohol (PVA) matrix (Rippey Corporation, E1 Dorado Hills, Ca, USA) as previously described (3). Capsules were sealed by photopolymerization of an acrylatebased glue. The encapsulated EBs were maintained at 37°C for five days in ES cell medium without LIF to allow for the attachment of the encapsulated cells to the capsule wall before implantation. Encapsulated glial precursor cells were maintained at 37°C for five days in N3EFL medium, to keep the precursors initially proliferating, which is considered to be an advantage when using capsules with matrix material.

### Adenosine release

The amount of adenosine released from ES cell-derived EBs was assessed from plated and encapsulated cells. ES cell-derived EBs were suspended in 10 ml of ES cell medium without LIF at a density of 1-2 x 105 cells/ml. For sample collection, the medium was replaced with fresh medium 24 hours after suspension and 2h later 300 ocl of medium was collected. These aliquots were analyzed in an enzyme-coupled bioluminescence assay as described (Fedele DE, Koch P, Brüstle O, et al. Engineering embryonic stem cell derived glia for adenosine delivery. Neurosci Lett 2004;370:160-165.). To analyze the amount of adenosine released from encapsulated ES cellderived EBs, four capsules of each genotype were made and incubated in pairs in 500µL of medium. 2 hours after changing the medium, 200µL of the culturing medium was collected for adenosine analysis.

### Animals and surgery

All animal experiments were conducted in accordance to guidelines of the local animal welfare authorities. Male Sprague-Dawley rats (Harlan Netherland, Horst, The Netherlands) were obtained at a body weight of 220 to 240 g. All rats were allowed to become acclimated for at least one week before being used in the experiments. The rats were housed (two rats per cage) under 12 h light / dark cycle (lights on from 8:00 A.M) with food and water provided *ad libitum.* Under general pentobarbital anesthesia (50 mg/kg, i.p.), rats (n = 20) were placed in a Kopf stereotactic frame, and bipolar, coated, stainless-steel electrodes (0.20 mm in diameter, Bilaney Consultants, Düsseldorf, Germany) were implanted bilaterally into the hippocampus and fixed with a pedestal of dental acrylate. Coordinates for hippocampal electrodes were (toothbar at 0): 5.0mm caudal to bregma, 4.8 mm lateral to midline, and 7.0 mm ventral to dura.

### Kindling and cell grafting

One week after electrode implantation, the animals were stimulated unilaterally up to 12 times daily with a Grass S-88 stimulator (1-ms square-wave pulses of 50 V at 10-Hz frequency for 10 s, 5-min interval between stimulations). Stimulations were maintained for a total period of up to 15 days until all animals reacted reproducibly with a grade 5 seizure after the first daily test stimulus. Behavioral seizures were scored according to the scale of Racine (Racine R. Kindling: the first decade. Neurosurg 1978;3:234-252.). Using this strict kindling paradigm about 30 % of kindled rats did not respond to conventional antiepileptic drugs (Huber A, Padrun V, Deglon N, Aebischer P, Mohler H, Boison D. Grafts of adenosine-releasing cells suppress seizures in kindling epilepsy. Proc Natl Acad Sci USA 2001;98:7611-7616.). These pharmacoresistant animals were identified by resistance to phenytoin (in 50% polyethylenglycol 400 in saline; 60 mg/kg i.p.) and were excluded from this study to be used in an ongoing project on pharmacoresistance in the rat kindling model of epilepsy.

Under stereotactic guidance *Adk*-/- or wild-type *Adk*+/+ ES cell-derived implants were grafted into the lateral brain ventricle of kindled rats ipsilateral to the site of stimulation. Coordinates for capsule implantation were (toothbar at 8.3): 1.8 mm caudal to bregma, 1.4 mm lateral to midline, and 8.5 mm ventral to dura.

### Assessment of seizure suppression

Beginning three days after grafting, the anticonvulsant efficacy of encapsulated *Adk*-/- ES cellderived implants or their wild-type *Adk*+/+ counterparts was tested by the delivery of intrahippocampal test stimulations (1 ms square-wave pulses of 50 V at 10 Hz frequency for 10 s) every other day. Convulsions were visually scored according to the scale of Racine (Racine R. Kindling: the first decade. Neurosurg 1978;3:234-252.). The electroencephalogram (EEG) was recorded for periods of 1 min before and 4 min after application of the stimulating pulse using a Human Scoring registration/software unit (B. Geehring, Institute of Pharmacology and Toxicology, University of Zurich).

### Histology

Two and seven days after grafting, rats containing *Adk*+/+ and *Adk-*/*-* EB derived implants were killed and their capsules (n = 2 - 5 each) retrieved. Capsules were fixed in 4% paraformaldehyde (EMS, Hatfield, PA) with 1% glutaraldehyde (Fluka, Buchs, Switzerland) in PBS for three hours, rinsed in PBS, and dehydrated through graded alcohol washes. In addition, to check for the *in vitro* viability of encapsulated cells, two capsules containing EBs from each genotype were fixed 12 days after encapsulation corresponding to day 7 of the *in vivo* study. To cut micrometer sections, the capsules were embedded with a commercial glycol methacrylate kit (Fluka). Procedures were performed according to the manufacturer. After hardening of the embedded capsules at 60°C for three days, sections of 1.0 µm thickness (Supercut microtome 2065; Reichert) were mounted on glass slides and stained with hematoxylin (Papanicolaou, Merck, Darmstadt, Germany) and eosin (Fluka). After retrieval of capsules from rat brain, 25µm coronal slices of the host brain were cut on a microtome and stained with cresyl violet as described previously (Boison D, Scheurer L, Tseng JL, Aebischer P, Mohler H. Seizure suppression in kindled rats by intraventricular grafting of an adenosine releasing synthetic polymer. Exp Neurol 1999;160:164-174.) and analyzed for implant location and surrounding tissue reactions.

### RESULTS

### Adenosine release by Adk-/- EBs

In a previous study the amounts of adenosine released from undifferentiated ES cells and from glial precursor cells were determined (Fedele DE, Koch P, Brüstle O, et al. Engineering embryonic stem cell derived glia for adenosine delivery. Neurosci Lett 2004;370:160-165.). In that study, only minimal amounts of adenosine were released from *Adk-*/*-* ES cells (2.6 ± 0.4 ng adenosine per 105 cells per hour), while undifferentiated *Adk*-/- N3EFL glial precursor cells released 11.7 ± 1.7 ng adenosine per 105 cells per hour. Since embryoid bodies represent an intermediate step in the differentiation of ES cells to glial precursors and, therefore, constitute a potentially versatile tool for cell encapsulation, in the present study the amounts of adenosine released by plated and encapsulated *Adk*+/+ and *Adk-*/- ES cell-derived EBs were analyzed by an enzymecoupled bioluminescent assay. 2 hours after changing the medium in which floating Ebs were cultured, the adenosine concentration in the medium from *Adk*-/- EBs (n = 5) was found to be 21.6 ± 13.2 ng/ml while comparable medium from *Adk*+/+ EBs (n = 5) was found to contain 4.5 ± 3.3 ng/mL adenosine (Fig. 10). Encapsulated ES cell-derived EBs (n = 4 for each genotype) were cultured in pairs in 500uL medium and 2 hours after changing the medium a sample was collected for adenosine analysis. The amount of adenosine found in the medium from encapsulated *Adk*-/- EBs was 19.4 ± 13.2 ng/ml compared to 3.1 ± 0.5 ng/ml adenosine in medium from encapsulated *Adk*+/+ EBs (Fig. 10). Therefore, EBs derived from *Adk*-/- ES cells release significant amounts of adenosine in culture and when encapsulated.

### Seizure suppression by adenosine-releasing stem cells

To assess the potential for seizure suppression, *Adk*-/- ES cells were *(i)* differentiated into Ebs and glial precursor cells, *(ii)* encapsulated into semipermeable membranes to study the paracrine effect of adenosine isolated from effects due to cellular engraftment and *(iii)* subsequently tested for seizure suppression after grafting of a single capsule into the lateral brain ventricle of kindled rats (n = 5 for each cell type). In parallel, a control group of kindled rats was implanted with capsules containing wild-type *Adk*+/+ EBs and glial precursor cells (n = 5 for each cell type). Three days before implantation all kindled animals reproducibly responded with a convulsive grade 5 seizure to every test stimulus (n = 20) (Fig. 11). Three, five and seven days after implantation of a capsule, the rats were subjected to single test stimuli and the behavioral seizure responses were scored. The experiment was terminated at day seven. In kindled animals that had received control *Adk*+/+ EB and glial precursor cell implants (n = 5 for each cell type), convulsive grade 5 seizure activity was not reduced as a result of grafting (Fig. 11). In contrast, three days after the implantation of *Adk*-/- EB and glial precursor cell implants, all animals (n = 5 for each cell type) displayed complete protection from any behavioral seizure activity (grade 0) (Fig. 11). Thus *Adk*-/- EB as well as *Adk*-/- glial precursor cell implants released amounts of adenosine sufficient to suppress kindled seizures. Control animals implanted with wild type *Adk*+/+ EB or glial precursor cell grafts continued to react with convulsive grade 5 seizures after each test stimulus (Fig. 11). Five days after implantation of the capsules, two rats grafted with *Adk*-/- EB implants and three animals grafted with *Adk*-/- glial precursor cell implants were still completely protected from seizure activity (grade 0) and the remaining three and two rats in the respective groups displayed preimplantation grade 5 seizures (Fig. 11). Seven days after grafting of *Adk*-/- ES cell-derived implants, all animals had returned to preimplantation grade 5 seizure activity (Fig. 11). It is important to note that the anticonvulsive action of the encapsulated adenosine releasing stem cell-derived cells led to an "all-or-nothing" effect, since either complete seizure suppression (grade 0) or, as in the controls, maximal grade 5 seizure activity was observed. Intermediate seizure grades were not observed.

### Suppression of afterdischarge activity by adenosine-releasing stem cells.

The antiepileptic effect of *Adk*-/- ES cell-derived implants was further investigated by analyzing epileptic afterdischarges in bilateral intrahippocampal EEG measurements. During a 10 second stimulus of 50 V at 10 Hz a stimulation artifact is observed in the EEG, which is variable from rat to rat, but is unchanged before and after implantation of the capsule within the same animal (Fig. 12). After the stimulation artifact the responding epileptic discharge of hippocampal neurons is observed as high frequency, rhythmic spikes ipsalateral and contralateral to the stimulus (Huber A, Padrun V, Deglon N, Aebischer P, Mohler H, Boison D. Grafts of adenosine-releasing cells suppress seizures in kindling epilepsy. Proc Natl Acad Sci USA 2001;98:7611-7616.). Three days before grafting, strong electric afterdischarges that accompanied stimulus-elicited grade 5 seizures were recorded in all rats used in this study (Fig. 12A, B, C and D, *first panel*). Control *Adk*+/+ EB and glial precursor cell implants had no influence on this epileptic afterdischarge activity as shown in EEG recordings taken five days after implantation (Fig. 12A and C, *second panel*). In these animals grade 5 seizures were consistently elicited by each test stimulus. In contrast, three and five days after the implantation of adenosine-releasing *Ask*-/- EB and glial precursor cell implants, seizure suppression was paralleled by a suppression of afterdischarge activity as became evident in EEG recordings from protected rats (Fig. 12B and D, *second panel*). It is important to note that suppression of afterdischarge activity always correlated with seizure suppression (grade 0). Thus, behavioral seizure suppression was associated with a reduction of epileptic afterdischarge activity.

### Seizure suppression is limited by cell viability within the capsules

To assess the viability of cells within the capsules, a histological analysis was done on capsules taken from rats at various time points after implantation. Capsules loaded with ES cell-derived EBs and taken from rats at 2 and 7 days after implantation were fixed and stained with hematoxylin-eosin. Viable cells were observed in capsules taken at 2 days after implantation by the positive purple stain of intact nuclei with hematoxylin and pink-stained cytoplasm with eosin (Fig 13). However, in capsules taken 7 days after implantation cell debris could be observed but no purple nuclei, indicating that the cells were dead at this time point (Fig 13). Thus, as shown earlier (Huber A, Padrun V, Deglon N, Aebischer P, Mohler H, Boison D. Grafts of adenosine-releasing cells suppress seizures in kindling epilepsy. Proc Natl Acad Sci USA 2001;98:7611-7616.), the loss of the antiseizure efficacy of grafted *Adk*-/- implants over time was found to correlate with cell loss within the capsules. In contrast, EB-derived cell capsules maintained *in vitro* for the same time period did not show any significant cell loss. Cresyl violet stained coronal brain sections of graft recipients did not show any adverse tissue reactions surrounding the implant site (data not shown).

### Example 8. Functional Properties of ES cell-derived neurons engrafted into the hippocampus of adult normal and chronically epileptic rats

The following experiments were performed to assess the potential of ES cell-derived neural precursors for functional integration following transplantation into the hippocampus of adult control and chronically epilepticrats.

### Methods

*Pilocarpine model*: Pilocarpine treatment of rats was carried out largely as described previously (Wellmer J, Su H, Beck H, Yaari Y. Long-lasting modification of intrinsic discharge properties in subicular neurons following status epilepticus. Eur J Neurosci 2002;16:259-266.). Briefly, male wistar rats (170 - 180 g) were injected subcutaneously (s.c.) with 370 mg / kg pilocarpine 30 min following pretreatment with 1.1 mg / kg scopolamine s.c. in order to reduce peripheral cholinergic side effects. Following pilocarpine injection, a majority of the animals developed a limbic status epilepticus that was terminated by injection of diazepam 40 min after onset (0.5 - 2.5 mg). In the minority of animals that did not develop status epilepticus after the first injection of pilocarpine, a second, identical s.c. injection of pilocarpine was administered. The rats were tended and fed with glucose-solution for one day and kept in separate cages. Sham-control animals were treated in an identical manner, but were injected with saline instead of pilocarpine. Characteristic seizure-related changes in the pilocarpine-treated animals were assessed by the macroscopic hippocampal atrophy as well as by TIMMstains that visualize the zinc-rich mossy fibers, and thus the characteristic mossy fiber sprouting.

*ES cells*: In this study, ES cell-derived neural precursors were used for transplantation that were genetically engineered to express enhanced green fluorescent protein (EGFP) under control of the tau promoter (Tucker KL, Meyer M, Barde YA. Neurotrophins are required for nerve growth during development. Nat Neurosci 2001;4:29-37.). These cells are derived from the J1 ES cell line (Li E, Bestor TH, Jaenisch R. Targeted mutation of the DNA methyltransferase gene results in embryonic lethality. Cell 1992;69:915-926.) and carry the cDNA for EGFP targeted in-frame into exon 1 of the tau gene. This results in a fusion protein consisting of the first 31 amino acids of tau and EGFP. The generation of ES cell-derived neural precursors from tau EGFP knock-in ES cells was performed as described previously (Okabe S, Forsberg-Nilsson K, Spiro AC, Segal M, McKay RD. Development of neuronal precursor cells and functional postmitotic neurons from embryonic stem cells in vitro. Mech Dev 1996;59:89-102; Wernig M, Tucker KL, Gornik V, et al. Tau EGFP embryonic stem cells: an efficient tool for neuronal lineage selection and transplantation. JNeurosci Res 2002;69:918-924.). Briefly, ES cells were aggregated to embryoid bodies, which were subsequently plated and propagated in ITSFn medium (DMEM-F12 supplemented with 5 µg/ml insulin, 50 µg/ml transferrin, 30 nM sodium selenite, and 5 µg/ml fibronectin) for 5 - 7 d. Cells were then trypsinized, triturated to a single-cell suspension, and replated in polyornithine-coated dishes. They were then propagated for an additional 4 - 5 d in DMEM-F12 supplemented with 25 µg/ml insulin, 50 µg/ml transferrin, 30 nM sodium selenite, 20 nM progesterone, 100 nM putrescine, 1 µg/ml laminin, and 10 ng/ml fibroblast growth factor 2. Media, supplements, and growth factors were obtained from Invitrogen (Karlsruhe, Germany), R & D Systems (Wiesbaden, Germany) and Sigma (Taufkirchen, Germany). For transplantation, donor cells were trypsinized and triturated through flame-polished Pasteur pipettes. They were then washed in calcium- and magnesium-free HBSS and concentrated to 5 - 8 x 104 cells/µl. After in vitro differentiation of this cell line, EGFP fluorescence has been found to be restricted to neuronal progeny (Wernig et al., *supra*).

*Transplantation*: One month after pilocarpine-treatment, the rats were anesthetised with intraperitoneal injection of ketamine hydrochloride (8 mg / 100 g) and xylazine hydrochloride (1 mg / 100 g), and placed in a stereotactic frame (Stoelting, Wood Dale, USA) for intrahippocampal transplantation of ESNs. Two µl of a cell suspension containing approximately 150.000 ES cell-derived neural precursors derived as described above, with 0.1% DNAse, were injected bilaterally into the hippocampi of 7 control and 11 pilocarpine-treated rats via a thin glass capillary. The animals were immunosuppressed with daily intraperitoneal injections of cyclosporine (1 mg / 100 g body weight; Novartis, Basel, Switzerland). Macroscopically, teratoma formation was never observed. Care and use of the animals conformed to institutional policies and guidelines.

*Electrophysiology:* Electrophysiological recordings were performed 13 to 34 days after transplantation. Rats were anesthetised with ether and subsequent injection of ketamine hydrochloride and xylazine hydrochloride. After thoracotomy, the descending aorta was clamped and the animals were perfused via the left ventricle with 40 to 50 ml of ice-cold solution of the following composition (in mM): 60 NaCl, 100 sucrose, 2.5 KCl, 1.25 NaH2PO4, 26 NaHCO3, 1 CaCl2, 5 MgCl2, 25 glucose. Following perfusion, rats were decapitated, the brain rapidly removed, and 300 µm coronal slices were cut with a vibratome. After cutting, slices were stored for 30 min at 36°C in the sucrose-containing solution used for heart perfusion. Subsequently, they were maintained in artificial cerebrospinal fluid (ACSF) of the following composition (in mM): 135 NaCl, 3 KCl, 1.25 NaH2PO4, 25 NaHCO3, 2 CaCl2, 1 MgCl2, 25 glucose (pH 7.4, room temperature). For whole-cell patch-clamp recordings, one slice at a time was transferred onto the stage of an upright microscope (Axioskop FS 11, Zeiss, Jena, Germany) equipped for infrared differential interference contrast and fluorescence, and perfused with ACSF (0.7 ml / min). Embryonic stem cell-derived neurons (ESNs) were identified by their expression of EGFP, which resulted in a strong fluorescence signal that was readily identified using a fluorescence camera (Spot Jr., Diagnostic Instruments / Visitron Systems, Puchheim, Germany). EGFP+ cells were subsequently visualized using infrared video microscopy and differential interference contrast optics to obtain patch-clamp recordings under visual control. Whole-cell voltage- and current-clamp recordings from ESNs were obtained using the patch-clamp technique. Borosilicate capillaries (4 - 5.5 MΩ) were fabricated with a vertical puller (PP-830, Narishige, Tokyo, Japan) and filled with an intracellular solution containing (in mM): 87.5 Cs-methanesulfonate, 10 N-(2- hydroxyethyl)piperazine-N'-(2-ethanesulfonate) (HEPES), 20 tetraethylammonium chloride, 5 1,2-Bis(2-aminophenoxy)ethane-N,N,N',N'-tetraacetic acid (BAPTA), 10 glucose, 5 MgCl2, 0.5 CaC12, 10 Na2-ATP, 0.5 GTP, 5 Lidocaine N-ethyl bromide, 20 sucrose, 0.1% biocytin (pH 7.2) for recordings of postsynaptic currents, otherwise they contained (in mM): 20 KCl, 130 K-gluconate, 1 Ethylene glycol-bis(2-aminoethylether)- N,N,N',N'-tetraacetic acid (EGTA), 10 HEPES, 0.35 CaCl2, 2 MgCl2, 2 Na2-ATP, 0.1% biocytin (pH 7.2). After obtaining the whole-cell configuration, voltage- and current-clamp recordings were carried out with an EPC-9 amplifier (HEKA Elektronik, Lambrecht, Germany). Data were sampled at 5 to 50 kHz (voltage-clamp recordings) and 4 to 25 kHz (current-clamp recordings), and filtered appropriately. In voltage-clamp recordings, series resistance compensation was employed to minimize voltage errors (average series resistance compensation 60%, average series resistance 13 MΩ). For pharmacological isolation of postsynaptic GABAA receptor-mediated currents, 50 µM 6- cyano-7-nitroquinoxaline-2,3-dione disodium salt (CNQX) and 50 µM DL-2-amino-5-phosphonopentanoic acid (DL-AP5) were added to the ACSF. Non-NMDA receptormediated excitatory postsynaptic currents were isolated by adding 50 µm DL-AP5 and 10 µM bicuculline to the ACSF. All recordings were performed at room temperature. Apart from CNQX and DL-AP5 (Tocris, Ellisville, USA) all chemicals were obtained from Sigma. Student's t-test (significance level 0.05) or nonparametric tests were used for statistical analysies. All data are given as means ± SEM.

*Immunohistochemistry*: After electrophysiology, slices were fixed in 4% paraformaldehyde phosphate buffered saline (PBS; Seromed, Berlin, Germany) for 1 to 2 days. Part of the slices were processed for fluorescent Texas red avidin (Dako, Hamburg, Germany) or permanent diaminobenzidine (DAB) biocytin staining according to standard techniques. From the remaining slices, 25 - 30 µm cryostat sections were obtained after cryopreservation in 30% sucrose saline. Double immunofluorescence stainings for EGFP and M6, a mouse-specific membrane-bound protein, were done according to established protocols. Briefly, cryostat sections were pretreated with 5% normal goat serum (NGS; Sigma) in PBS for 30 min. Then, slices were incubated overnight with PBS containing 0.1% sodium azide, 3% NGS, and antibodies for M6 (1:10; Developmental studies hybridoma bank, Iowa City, USA) and EGFP (1:1000; Dako). The next day, the slices were washed with PBS and secondary antibodies for EGFP (FITC anti rabbit IgG, 1:200; Jackson, West Grove, USA) and M6 (biotin goat anti rat, 1:50; Jackson) in PBS with 3% NGS were applied for 2 - 4 hours. Finally, after washing with PBS, Texas Red avidin (1:125) in 10 mM HEPES buffer (pH 7.5) was applied for 1.5 hours.

### Results

Following transplantation, ESNs were readily identified by their expression of EGFP. This allowed detection of intrahippocampal transplants in 4 sham-control and 5 pilocarpine-treated rats. The locations of grafts are summarized in Fig. 14A. Double immunofluorescence staining for both EGFP and M6, a mouse-specific membranebound protein, confirmed that a large fraction of transplanted ES cell-derived neural precursors give rise to EGFP+ ESNs (Fig. 14B and C depict representative examples from control and pilocarpine-treated animals, respectively). In both control and epileptic animals, graft-derived ESNs generally remained in clusters at the transplant sites or close to injection canals. Upon closer examination, the borders of the clusters were not sharply delineated, with individual ESNs migrating short distances into the host tissue.

Contrasting with the lack of significant migration into host tissue, grafted ESNs showed a striking propensity to extend processes into host brain tissue. EGFP+/M6 immunoreactive processes were observed tens to hundreds of µm distant from the transplant site within host tissue. A representative example of this phenomenon is depicted in Figure 15. In this case, the transplant was situated immediately adjacent to the CA1 region (transplant shown as EGFP/M6 immunolabeling superimposed on a phase-contrast image of the hippocampal slice). A dense plexus of EGFP/M6 double labeled fibers was observed at considerable distances from the transplant site, both within the corpus callosum, the CA1 and the CA3 region (Fig. 15, panels A-C).

### Intrinsic membrane properties of graft-derived ESNs

EGFP+ cells were identified using fluorescence microscopy (Fig. 16A11), and subsequently obtained patch-clamp recordings under visual control using infrared differential interference contrast optics. ESNs that had migrated away from the transplant sites or were situated at transplant borders were recorded from preferentially. The intrinsic membrane properties of ESNs were examined initially. There were no significant differences in the passive membrane properties of ESNs in control (capacitance 47.6 ± 3.6 pF, n = 13; membrane resistance 1.46 ± 0.37 GΩ, n = 10) versus pilocarpine-treated rats (capacitance 40.0 ± 4.8 pF, n = 13; membrane resistance 1.51 ± 0.38 GΩ, n = 10; see table 1). In both groups, 80 to 90% of ESNs displayed spontaneous rhythmic action potential discharges at frequencies of 2.9 ± 0.6 Hz (n = 8) and 3.4 ± 0.7 Hz (n = 9) in control and epileptic animals, respectively (not significant, Fig. 15B1). For further analysis of action potential morphology, ESNs were clamped to hyperpolarized membrane potentials of around -75 mV by current injection in order to suppress the spontaneous discharges. All ESNs were able to generate single action potentials in response to brief current injections of 3 ms duration. From these recordings, the action potential threshold was derived, amplitude and half-width. Plotting these values versus the time following transplantation revealed a tendency for action potentials to become narrower and larger with time after transplantation (Fig. 16B2, see panel C for summary and table 1 for average values of all measurements, as well as comparison to host granule neurons). Current injections of 250 ms and 1 s duration resulted in trains of action potentials with varying amounts of spike frequency adaptation. Likewise, ESNs showed variable fast (compare upper and lower traces in Fig. 16B3) and, sometimes, slow afterhyperpolarizations. Few ESNs also showed spike afterdepolarizations. Spike afterpotentials were heterogeneous in both pilocarpine-treated and control rats, with no obvious group differences.

**Table 1**

| | **membrane capacitance [pF]** | **input resistance [GΩ]** | **threshold [mV]** | **amplitude [mV]** | **half-width [ms]** |
|---|---|---|---|---|---|
| **control** | 47.6 ± 3.6 (n = 13) | 1.46 ± 0.37 (n=10) | -49.8 ± 1.6 (n = 9) | 52.6 ± 2.4 (n = 9) | 2.29 ± 0.13 (n = 9) |
| **pilocarpinetreated** | 40.0 ± 4.6 (n = 13) | 1.51 ± 0.38 (n = 10) | -51.1 ± 2.5 (n = 10) | 48.4 ± 2.9 (n = 10) | 2.74 ± 0.19 (n = 10) |
| **endogenous granule cells control (n = 4)** | 120.7 ± 10.5 | 0.248 ± 0.039 | -57.0 ± 3.9 | 107.0 ± 3.1 | 1.20 ± 0.02 |
| **endogenous granule cells pilocarpine-treated (n = 3)** | 130.6 ± 19.0 | 0.308 ± 0.064 | -55.3 ± 1.9 | 104.3 ± 2.4 | 1.16 ± 0.03 |

Finally, more than 90% of the ESNs displayed inward rectification in response to hyperpolarizing current injections (Fig. 16B3). Following hyperpolarizing current injection, part of the ESNs exhibited rebound action potential discharges (44 % and 17 % of ESNs in control and pilocarpine-treated rats, respectively, n.s., Fisher's exact test). Voltage-clamp recordings revealed voltage-dependent membrane currents in all ESNs. Depolarizing voltage steps induced large, unclamped inward currents corresponding to voltage-gated Na+ channels. Furthermore, outward K+ currents were present in all ESNs, similar to voltage-gated ionic currents described in ESNs transplanted into hippocampal slice cultures (10, data not shown).

### Synaptic input onto ESNs

More than 80% of ESNs received spontaneous synaptic input (Fig. 17). Excitatory non- NMDA and inhibitory GABAA receptor-mediated components were isolated pharmacologically. Addition of 50 µM DL-AP5 and 10 µM bicuculline to the ACSF yielded non-NMDA receptor-mediated postsynaptic currents with frequencies of 0.7 ± 0.3 Hz (n = 7) in control and 1.2 ± 0.5 Hz (n = 8) in epileptic rats. GABAA receptormediated currents were isolated by adding 50 µM CNQX and 50 µM DL-AP5 to the ACSF. They had frequencies of 4.6 ± 0.9 Hz (n = 4) and 3.2 ± 0.5 Hz (n = 5) in control and epileptic rats, respectively.

In subsequent experiments, postsynaptic currents were elicited by stimulation with a monopolar stimulation electrode placed in the vicinity of the recorded cell. Stimulation from more distant sites proved to be difficult and was possible only in some cases. The non-NMDA receptor-mediated postsynaptic currents displayed a reversal potential of - 2.2 ±1.4 mV (control rats, n = 4) and 0.5 ± 5.8 mV (pilocarpine-treated rats, n = 6) with decay time constants of 8.9 ± 1.9 ms (n = 4) and 6.8 ± 1.0 ms (n = 6), respectively (Fig. 18A and C). In all tested ESNs (n = 4 and 5 for control and epileptic animals, respectively), these currents were completely suppressed by additional perfusion of 50 µM CNQX (Fig. 18B).

GABAA receptor-mediated synaptic currents displayed a slower decay than the excitatory postsynaptic currents (23.4 ± 5.1 ms, n = 4, control rats and 24.5 ± 4.4, n = 5, pilocarpine-treated rats). Their reversal potentials were -47.9 ± 2.2 mV (n = 4) and -43.9 ± 1.4 mV (n = 5), in the control and epileptic groups, respectively (Fig. 19A and C). These values, as well as the reversal potentials obtained for glutamatergic EPSCs, reflect our artificial ionic conditions. In the case of GABAA receptors, as expected, these values are close to the calculated chloride reversal potential (-39 mV). The currents were completely suppressed by 10 µM bicuculline in 2 out of 2 ESNs from control and 4 out of 4 ESNs from pilocarpine-treated rats (Fig. 19B).

Next, short-term plasticity of these synapses was investigated by applying double pulses of different interpulse intervals ranging from 20 to 160 ms. As shown in Fig. 20, non-NMDA receptor-mediated currents displayed significant short-term facilitation at most interpulse intervals which was generally more pronounced at shorter intervals (Fig. 20A). Short-term plasticity was less pronounced for GABAA receptor-mediated currents (Fig. 20B).

No differences between ESNs transplanted into sham-control and pilocarpine-treated rats were observed for any parameter tested with regard to both intrinsic and synaptic properties.

### Example 9: Engrafting Adk^{-/-} ES cell derived neural precursor cells in the rat hippocampus

This example provides a description of the generation of *Adk*^{*-*/*-*} ES cells, their subsequent differentiation into pan-neural precursor cells, and implantation into the rat brain.

### Adk^{-/}⁻ ES cell preparation

ES cells bearing homozygous deletion of both alleles of the *Adk* gene may be generated by any of the methods described in the specification, but particularly accordingly to the actual experimental methods described in Example 1.

### Partial differentiation of Adk^{-/}⁻ ES cells

The generation of ES cell-derived neural precursors from *Adk*^{*-*/*-*} ES cells may be performed as described above. Briefly, ES cells are aggregated to embryoid bodies, which are subsequently plated and propagated in ITSFn medium (DMEM-F12 supplemented with 5 µg/ml insulin, 50 µg/ml transferrin, 30 nM sodium selenite, and 5 µg/ml fibronectin) for 5 - 7 d. Cells are then trypsinized, triturated to a single-cell suspension, and replated in polyornithine-coated dishes. They are then propagated for an additional 4 - 5 d in, for example, DMEM-F12 supplemented with 25 µg/ml insulin, 50 µg/ml transferrin, 30 nM sodium selenite, 20 nM progesterone, 100 nM putrescine, 1 µg/ml laminin, and 10 ng/ml fibroblast growth factor 2. Media, supplements, and growth factors may be obtained from, for example, Invitrogen (Karlsruhe, Germany), R & D Systems (Wiesbaden, Germany) and Sigma (Taufkirchen, Germany). For transplantation, donor cells are trypsinized and triturated through flame-polished Pasteur pipettes. They are then washed in calcium- and magnesium-free HBSS and concentrated to 5 - 8 x 10⁴ cells/µl.

### Transplantation:

In order to test the ability of the differentiated *Adk*^{*-*/*-*} ES cells to serve as a useful treatment for epilepsy, the cells are transplanted into a rat model of epilepsy: the pilocarpine model. Pilocarpine treatment of rats can be carried out largely as described previously (Wellmer J, Su H, Beck H, Yaari Y. Long-lasting modification of intrinsic discharge properties in subicular neurons following status epilepticus. Eur J Neurosci 2002; 16:259-266.). Briefly, male wistar rats (170 -180 g) are injected subcutaneously (s.c.) with 370 mg / kg pilocarpine 30 min following pretreatment with 1.1 mg / kg scopolamine s.c. in order to reduce peripheral cholinergic side effects. Following pilocarpine injection, a majority of the animals should develop a limbic status epilepticus that can be terminated by injection of diazepam 40 min after onset (0.5 - 2.5 mg). In the minority of animals that do not develop status epilepticus after the first injection of pilocarpine, a second, identical s.c. injection of pilocarpine is administered. The rats should be tended and fed with glucose-solution for one day and kept in separate cages. Sham-control animals are treated in an identical manner, but are injected with saline instead of pilocarpine. Characteristic seizure-related changes in the pilocarpine-treated animals can be assessed by the macroscopic hippocampal atrophy as well as by TIMMstains that visualize the zinc-rich mossy fibers, and thus the characteristic mossy fiber sprouting.

One month after pilocarpine-treatment, rats are anesthetised with intraperitoneal injection of ketamine hydrochloride (8 mg /100 g) and xylazine hydrochloride (1 mg / 100 g), and placed in a stereotactic frame (Stoelting, Wood Dale, USA) for intrahippocampal transplantation of ESNs. Two µl of a cell suspension containing approximately 150.000 ES cell-derived neural precursors derived as described above, with 0.1% DNAse, is injected bilaterally into the hippocampi of control and pilocarpine-treated rats via a thin glass capillary. The animals are immunosuppressed with daily intraperitoneal injections of cyclosporine (1 mg / 100 g body weight; Novartis, Basel, Switzerland). Macroscopically, teratoma formation should not be observed.

The transplants, although unlikely to migrate significantly into host tissue, will develop a dense network of processes extending over large distances into the host tissue. The transplanted cells possess the ability to generate action potentials and to express voltage-gated sodium and potassium currents, as well as hyperpolarization-activated currents. Most of the transplanted cells will establish non-NMDA and GABA_{A} receptor-mediated synaptic input, both of which are capable of displaying intact short-term plasticity.

The transplanted *Adk*^{*-*/*-*} cells are capable of releasing between about 2 and 20 ng adenosine per 10⁵ cells per hour, between 5 and 15 ng adenosine, and between about 10 and 15 ng or greater of adenosine per 10⁵ cells per hour. Rats receiving adenosine releasing *Adk*^{*-*/*-*}ES cell-derived implants should display at least transient protection from convulsive seizures and a reduction of afterdischarge activity in EEG recordings. Given the viability of the . implanted cells, however, it is likely that more long-term seizure suppression will be observed.

### References

Bjorklund LM, Sanchez-Pernaute R, Chung S, Andersson T, Chen IY, McNaught KS, Brownell AL, Jenkins BG, Wahlestedt C, Kim KS and Isacson O (2002) Embryonic stem cells develop into functional dopaminergic neurons after transplantation in a Parkinson rat model. Proc Natl Acad Sci USA 99:2344-2349.
Boison D, Huber A, Padrun V, Déglon N, Aebischer P and Möhler H (2002a) Seizure suppression by adenosine releasing cells is independent of seizure frequency. Epilepsia 43:788-796.
Boison D, Scheurer L, Tseng JL, Aebischer P and Mohler H (1999) Seizure suppression in kindled rats by intraventricular grafting of an adenosine releasing synthetic polymer. Exp Neurol 160:164-174.
Boison D, Scheurer L, Zumsteg V, Rülicke T, Litynski P, Fowler B, Brandner S and Mohler H (2002b) Neonatal hepatic steatosis by disruption of the adenosine kinase gene. Proc Natl Acad Sci USA 99:6985-6990.
Bontemps F, Van den Berghe G and Hers HG (1983) Evidence for a substrate cycle between AMP and adenosine in isolated hepatocytes. Proc Natl Acad Sci USA 80:2829-2833.
Brüstle O, Jones KN, Learish RD, Karram K, Choudhary K, Wiestler OD, Duncan ID and McKay RD (1999) Embryonic stem cell-derived glial precursors: a source of myelinating transplants. Science 285:754-756.
Brüstle O, Spiro AC, Karram K, Choudhary K, Okabe S and McKay RD (1997) In vitro-generated neural precursors participate in mammalian brain development. Proc Natl Acad Sci U S A 94:14809-14814.
Dunwiddie TV and Masino SA (2001) The role and regulation of adenosine in the central nervous system. Annu Rev Neurosci 24:31-55.
During MJ and Spencer DD (1992) Adenosine: a potential mediator of seizure arrest and postictal refractoriness. Ann Neurol. 32:618-624.
Fredholm BB (1997) Adenosine and neuroprotection. Int Rev Neurobiol 40:259-280.
Fredholm BB, AP IJ, Jacobson KA, Klotz KN and Linden J (2001) International Union of Pharmacology. XXV. Nomenclature and classification of adenosine receptors. Pharmacol Rev 53:527-552.
Gouder N, Fritschy JM and Boison D (2003) Seizure suppression by adenosine A1 receptor activation in a mouse model of pharmacoresistant epilepsy. Epilepsia 44:877-885.
Gouder N, Scheurer L, Fritschy J-M and Boison D (2004) Overexpression of adenosine kinase in epileptic hippocampus contributes to epileptogenesis. JNeurosci 24:692-701.
Huber A, Güttinger M, Möhler H and Boison D (2002) Seizure suppression by adenosine A2A receptor activation in a rat model of audiogenic brainstem epilepsy. Neurosci Lett 329:289-292.
Huber A, Padrun V, Déglon N, Aebischer P, Möhler H and Boison D (2001) Grafts of adenosine releasing cells suppress seizures in kindling epilepsy. Proc Natl Acad Sci USA 98:7611-7616.
Jallon P (1997) The problem of intractability - the continuing need for new medical therapies in epilepsy. Epilepsia 38:S 37-S 42.
Jarvis MF, Mikusa J, Chu KL, Wismer CT, Honore P, Kowaluk EA and McGaraughty S (2002) Comparison of the ability of adenosine kinase inhibitors and adenosine receptor agonists to attenuate thermal hyperalgesia and reduce motor performance in rats. Pharmacol Biochem Behav 73:573-581.
Klug MG, Soonpaa MH, Koh GY and Field LJ (1996) Genetically selected cardiomyocytes from differentiating embronic stem cells form stable intracardiac grafts. J Clin Invest 98:216-224.
Kowaluk EA and Jarvis MF (2000) Therapeutic potential of adenosine kinase inhibitors. Expert Opin Investig Drugs 9:551-564.
Liu S, Qu Y, Stewart TJ, Howard MJ, Chakrabortty S, Holekamp TF and McDonald JW (2000) Embryonic stem cells differentiate into oligodendrocytes and myelinate in culture and after spinal cord transplantation. Proc Natl Acad Sci U S A 97:6126-6131.
Loscher W and Potschka H (2002) Role of multidrug transporters in pharmacoresistance to antiepileptic drugs. J Pharmacol Exp Ther 301:7-14.
McDonald JW, Liu XZ, Qu Y, Liu S, Mickey SK, Turetsky D, Gottlieb DI and Choi DW (1999) Transplanted embryonic stem cells survive, differentiate and promote recovery in injured rat spinal cord. Nat Med 5:1410-1412.
McNally T, Helfrich RJ, Cowart M, Dorwin SA, Meuth JL, Idler KB, Klute KA, Simmer RL, Kowaluk EA and Halbert DN (1997) Cloning and expression of the adenosine kinase gene from rat and human tissues. Biochem Biophys Res Commun 231:645-650.
Nord LD, Stolfi RL, Colofiore JR and Martin DS (1996) Correlation of retention of tumor methylmercaptopurine riboside-5'-phosphate with effectiveness in CD8F1 murine mammary tumor regression. Biochem Pharmacol 51:621-627.
Okabe S, Forsberg-Nilsson K, Spiro AC, Segal M and McKay RD (1996) Development of neuronal precursor cells and functional postmitotic neurons from embryonic stem cells in vitro. Mech Dev 59:89-102.
Pak MA, Haas HL, Decking UKM and Schrader J (1994) Inhibition of adenosine kinase increases endogenous adenosine and depresses neuronal activity in hippocampal slices. Neuropharmacol 33:1049-1053.
Reubinoff BE, Itsykson P, Turetsky T, Pera MF, Reinhartz E, Itzik A and Ben-Hur T (2001) Neural progenitors from human embryonic stem cells. Nat Biotechnol 19:1134-1140.
Reubinoff BE, Pera MF, Fong CY, Trounson A and Bongso A (2000) Embryonic stem cell lines from human blastocysts: somatic differentiation in vitro. Nat Biotechnol 18:399-404.
Sawynok J, Reid A and Poon A (1998) Peripheral antinociceptive effect of an adenosine kinase inhibitor, with augmentation by an adenosine deaminase inhibitor, in the rat formalin test. Pain 74:75-81.
Simpson EM, Linder CC, Sargent EE, Davisson MT, Mobraaten LE and Sharp JJ (1997) Genetic variation among 129 substrains and its importance for targeted mutagenesis in mice. Nat Genet 16:19-27.
Sokoloski JA and Sartorelli AC (1987) Inhibition of mannose incorporation into glycoproteins and dolichol-linked intermediates of Sarcoma 180 cells by 6-methylmercaptopurine ribonucleoside. Int J Cancer 39:764-768.
Sommerschild HT and Kirkeboen KA (2000) Adenosine and cardioprotection during ischaemia and reperfusion--an overview. Acta Anaesthesiol Scand 44:1038-1055.
Soria B, Roche E, Berna G, Leon-Quinto T, Reig JA and Martin F (2000) Insulin-secreting cells derived from embryonic stem cells normalize glycemia in streptozotocin-induced diabetic mice. Diabetes 49:157-162.
Stevens B, Porta S, Haak LL, Gallo V and Fields RD (2002) Adenosine: a neuron-glial transmitter promoting myelination in the CNS in response to action potentials. Neuron 36:855-868.
Thomson JA, Itskovitz-Eldor J, Shapiro SS, Waknitz MA, Swiergiel JJ, Marshall VS and Jones JM (1998) Embryonic stem cell lines derived from human blastocysts. Science 282:1145-1147.
Thomson JA and Odorico JS (2000) Human embryonic stem cell and embryonic germ cell lines. Trends Biotechnol 18:53-57.
von Lubitz DKJE (1999) Adenosine and cerebral ischemia: therapeutic future or death of a brave concept? Eur J Pharmacol 365:9-25.
Wiles MV and Johansson BM (1999) Embryonic stem cell development in a chemically defined medium. Exp Cell Res 247:241-248.
Zhang SC, Wernig M, Duncan ID, Brüstle O and Thomson JA (2001) In vitro differentiation of transplantable neural precursors from human embryonic stem cells. Nat Biotechnol 19:1129-1133.
Zumsteg V and Boison D (2002) The use of real-time PCR with fluorogenic probes for the rapid selection of mutant neuroectodermal grafts. JNeurosci Meth 120:85-94.

### Uther Embodiments

Other embodiments will be evident to those of skill in the art. It should be understood that the foregoing detailed description is provided for clarity only and is merely exemplary. The and scope of the present invention are not limited to the above examples, but are encompassed by the following claims.
## Claims

1. An isolated human or mouse neural precursor cell deficient in the production of adenosine kinase, said cell comprising a defect in both alleles of the adenosine kinase gene, wherein said cell is able to differentiate into a neuron or glial cell,
obtainable by treating embryonic stem (ES) cells which have been isolated from a mammal, (a) so as to disrupt one allele of the adenosine kinase gene in said ES cell, thereby deriving an ES cell comprising a defect in one allele of an adenosine kinase gene;
treating said ES cell derived from step (a) so as to (b) inactivate the remaining allele of adenosine kinase gene, thereby generating an ES cell comprising a defect in both alleles of the adenosine kinase gene; and
differentiating said ES cells generated in step (b) to a neural precursor cell.

2. The neural precursor cell of claim 1 which releases adenosine at an increased amount as compared to a corresponding wild type cell.

3. An isolated glial cell deficient in production of adenosine kinase and differentiated from the isolated neural precursor cell of claim 1, said glial cell comprising a defect affecting both alleles of the adenosine kinase gene.

4. The glial cell of claim 4 releasing adenosine at an increased amount as compared to a corresponding wild type cell.

5. An isolated neuron deficient in production of adenosine kinase and differentiated from the isolated neural precursor cell of claim 1.

6. A composition comprising the isolated cell of claim 1, 2, 3, 4 or 5 and a polymer suitable for implantation into a mammal permitting release of adenosine.

7. The composition of claim 6, wherein the polymer permits release of adenosine by said cell.

8. The composition of claim 6 or 7, wherein the polymer prevents cell-cell contact between the mammal's immune system and the cells of the composition.

9. The composition of one of claims 6 to 8, comprising between 20,000 to 2,000,000 of cells.

10. The use of a neural precursor cell of claim 1 for the preparation of a medicament for releasing adenosine to a tissue wherein said medicament is to be implanted into said tissue site thereby permitting release of adenosine to a tissue.

11. The use of neural precursor cells of claim 1 for the preparation of a medicament for treating epilepsy in a subject, wherein the neural precursor cells release a chronic local dose of a therapeutic amount of adenosine, whereby said released adenosine reduces epileptic activity in the subject, and wherein said medicament is to be implanted into the brain of the subject.

12. The use of claim 11, wherein the neural precursor cells are encapsulated neural precursor cells deficient in the production of adenosine kinase and harboring a defect in both alleles of the adenosine kinase gene.

13. The use of claim 11 or 12, wherein the dose is in the range of 1 ng to 500 ng of adenosine per day.

14. The use of claim 12, wherein the encapsulated neural precursor cells comprises about 20,000 to about 2,000,000 neural precursor cells deficient in the production of adenosine kinase and comprising a defect in both alleles of the adenosine kinase gene.

15. The use of a neural precursor cell of claim 1 for the preparation of a medicament for treating epilepsy in a subject, wherein the neural precursor cells integrate into the brain and differentiate into neural cells, wherein the differentiated neural cells release a chronic local dose of therapeutic amount of adenosine, whereby said adenosine reduces epileptic activity in the subject, and wherein the medicament is to be implanted.

16. A method of producing a neural precursor cell deficient in the production of adenosine kinase and comprising a defect in both alleles of the adenosine kinase gene, comprising:
(a) treating embryonic stem (ES) cells which have been isolated from a mammal, so as to disrupt one allele of the adenosine kinase gene in said ES cell, thereby deriving an ES cell comprising a defect in one allele of an adenosine kinase gene;
(b) treating said ES cells derived in step (a) so as to inactivate the remaining allele of adenosine kinase gene, thereby generating an ES cell comprising a defect in both alleles of the adenosine kinase gene; and
differentiating said ES cells generated in step (b) to a neural precursor cell.

## Patentansprüche

1. Eine isolierte humane oder Maus- neurale Vorläufer-Zelle, die defizient im Bezug auf die Production von Adenosinkinase ist, wobei jene Zelle einen Defekt auf beiden Allelen des Adenosinkinase-Gens umfasst, wobei jene Zelle fähig ist, sich in ein Neuron oder eine Glia-Zelle auszudifferenzieren,
erreichbar durch Behandlung embryonaler Stamm (ES)-Zellen, die von einem Säuger isoliert wurde, (a) um ein Allel des Adenosinkinase-Gens in jener ES-Zelle zu unterbrechen, wodurch eine ES-Zelle erlangt wird, die eine Defekt in einem Allel eines Adenosinkinase-Gens umfasst;
Behandlung jener in Schritt (a) erlangten ES-Zelle, um (b) das verbleibende Adenosinkinase-Gen-Allel zu inaktivieren, wodurch eine ES-Zelle generiert wird, die einen Defekt auf beiden Allelen des Adenosinkinase-Gens umfasst; und
Differenzierung jener in Schritt (b) generierten ES-Zelle zu einer neuralen Vorläuferzelle.

2. Die neurale Vorläuferzelle von Anspruch 1, die Adenosin in einer höheren Menge im Vergleich zu einer entsprechenden Wildtyp-Zelle freisetzt.

3. Eine isolierte Gila-Zelle, die defizient im Bezug auf die Production von Adenosinkinase ist und sich aus der isolierten neuralen Vorläuferzelle von Anspruch 1 ausdifferenziert hat, wobei jene Gila-Zelle einen Defekt, der beide Allele des Adenosinkinase-Gens betrifft, umfasst.

4. Die Glia-Zelle von Anspruch 4, die Adenosin in einer höheren Menge im Vergleich zu einer entsprechenden Wildtyp-Zelle freisetzt.

5. Ein isoliertes Neuron, das defizient im Bezug auf die Produktion von Adenosinkinase ist und sich aus der isolierten neuralen Vorläuferzelle von Anspruch 1 ausdifferenziert hat.

6. Eine Zusammensetzung, umfassend die isolierte Zelle von Anspruch 1, 2, 3, 4 oder 5 und ein Polymer, das für Implantation in einen Säuger geeignet ist, was Adenosin-Freisetzung erlaubt.

7. Die Zusammensetzung von Anspruch 6, wobei das Polymer die Freisetzung von Adenosin durch jene Zelle erlaubt.

8. Die Zusammensetzung von Anspruch 6 oder 7, wobei das Polymer Zell-Zell-Kontakt zwischen dem Immunsystem des Säugers und den Zellen der Zusammensetzung verhindert.

9. Die Zusammensetzung von einem der Ansprüche 6 bis 8, umfassend zwischen 20.000 und 2.000.000 Zellen.

10. Die Verwendung einer neuralen Vorläuferzelle von Anspruch 1 zur Herstellung eines Medikaments für Freisetzung von Adenosin an ein Gewebe, wobei jenes Medikament an jene Gewebestelle zu implantieren ist, wodurch die Freisetzung von Adenosin an ein Gewebe erlaubt wird.

11. Die Verwendung von neuralen Vorläuferzellen von Anspruch 1 zur Herstellung eines Medikaments zur Behandlung von Epilepsie bei einem Subjekt, wobei die neuralen Vorläuferzellen eine chronische lokale Dosis einer therapeutischen Menge von Adenosin freisetzen, wodurch jenes freigesetzte Adenosin epileptische Aktivität bei dem Subjekt reduziert und wobei jenes Medikament in das Gehirn des Subjekt zu implantieren ist.

12. Die Verwendung von Anspruch 1, wobei die neuralen Vorläuferzellen eingekapselte neurale Vorläuferzellen sind, die defizient im Bezug auf die Produktion von Adenosinkinase sind und einen Defekt auf beiden Allelen des Adenosinkinase-Gens aufweisen.

13. Die Verwendung von Anspruch 11 oder 12, wobei die Dosis im Bereich von 1 ng bis 500 ng Adenosin pro Tag ist.

14. Die Verwendung von Anspruch 12, wobei die eingekapselten neuralen Vorläuferzellen etwa 20.000 bis etwa 2.000.000 neurale Vorläuferzellen umfassen, die defizient im Bezug auf die Produktion von Adenosinkinase sind und einen Defekt auf beiden Allelen des Adenosinkinase-Gens umfassen.

15. Die Verwendung einer neuralen Vorläuferzelle von Anspruch 1, zur Herstellung eines Medikaments zur Behandlung von Epilepsie bei einem Subjekt, wobei die neuralen Vorläuferzellen sich in das Gehirn integrieren und sich zu neuralen Zellen ausdifferenzieren, wobei die ausdifferenzierten neuralen Zellen eine chronische lolale Dosis einer therapeutischen Adenosin-Menge freisetzen, wodurch jenes Adenosin epileptische Aktivität in dem Subjekt reduziert und wobei das Medikament zu implantieren ist.

16. Ein Verfahren zur Herstellung einer neuralen Vorläuferzelle, die defizient im Bezug auf die Production von Adenosinkinase ist und einen Defekt auf beiden Allelen des Adenosinkinase-Gens umfasst, umfassend:
(a) Behandlung embryonaler Stamm (ES)-Zellen, die aus einem Säuger isoliert worden sind, um ein Allel des Adenosinkinase-Gens in jener ES-Zelle zu unterbrechen, wodurch eine ES-Zelle erlangt wird, die eine Defekt in einem Allel eines Adenosinkinase-Gens umfasst;
(b) Behandlung jener in Schritt (a) erlangten ES-Zelle, um das verbleibende Adenosinkinase-Gen-Allel zu inaktivieren, wodurch eine ES-Zelle generiert wird, die einen Defekt auf beiden Allelen des Adenosinkinase-Gens umfasst; und
Differenzierung jener in Schritt (b) generierten ES-Zelle zu einer neuralen Vorläuferzelle.

## Revendications

1. Cellule précurseur neuronale isolée humaine ou de souris déficiente en termes de production d'adénosine kinase, ladite cellule comprenant un défaut dans les deux allèles du gène d'adénosine kinase, dans laquelle ladite cellule est capable de se différencier en une cellule neuronale ou gliale,
pouvant être obtenue par traitement de cellules souches embryonnaires (ES) qui ont été isolées d'un mammifère, (a) de façon à interrompre un allèle sur le gène d'adénosine kinase dans ladite cellule ES, dérivant ainsi une cellule ES comprenant un défaut dans un allèle d'un gène d'adénosine kinase ;
par traitement de ladite cellule ES dérivée de l'étape (a) de façon à (b) inactiver l'allèle prestant du gène d'adénosine kinase, générant ainsi une cellule ES comprenant un défaut dans les deux allèles du gène d'adénosine kinase ; et
par différenciation desdites cellules ES générées dans l'étape (b) en une cellule précurseur neuronale.

2. Cellule précurseur neuronale selon la revendication 1, qui libère de l'adénosine en une quantité accrue par rapport à une cellule sauvage correspondante.

3. Cellule gliale isolée déficiente en termes de production d'adénosine kinase et différenciée à partir de la cellule précurseur neuronale isolée de la revendication 1, ladite cellule gliale comprenant un défaut affectant les deux allèles du gène d'adénosine kinase.

4. Cellule gliale selon la revendication 4 libérant de l'adénosine en une quantité accrue par rapport à une cellule sauvage correspondante.

5. Neurone insolé déficient en termes de production d'adénosine kinase et différencié à partir de la cellule précurseur neuronale isolée de la revendication 1.

6. Composition comprenant la cellule isolée de la revendication 1, 2, 3, 4 ou 5 et un polymère approprié pour une implantation dans un mammifère permettant la libération d'adénosine.

7. Composition selon la revendication 6, dans laquelle le polymère permet la libération d'adénosine par ladite cellule.

8. Composition selon la revendication 6 ou 7, dans laquelle le polymère empêche un contact de cellule à cellule entre le système immunitaire du mammifère et les cellules de la composition.

9. Composition selon l'une quelconque des revendications 6 à 8, comprenant entre 20 000 et 2 000 000 de cellules.

10. Utilisation d'une cellule précurseur neuronale selon la revendication 1, pour la préparation d'un médicament pour la libération d'adénosine dans un tissu, dans laquelle ledit médicament doit être implanté sur ledit site de tissu permettant ainsi la libération d'adénosine dans un tissu.

11. Utilisation de cellules précurseurs neuronales selon la revendication 1, pour la préparation d'un médicament pour le traitement de l'épilepsie chez un sujet, dans laquelle les cellules précurseurs neuronales libèrent une dose locale chronique d'une quantité thérapeutique d'adénosine, moyennant quoi ladite adénosine libérée réduise l'activité épileptique chez le sujet, et dans laquelle ledit médicament doit être implanté dans le cerveau du sujet.

12. Utilisation selon la revendication 11, dans laquelle les cellules précurseurs neuronales sont des cellules précurseurs neuronales encapsulées déficientes en termes de production d'adénosine kinase et logeant un défaut dans les deux allèles du gène d'adénosine kinase.

13. Utilisation selon la revendication 11 ou 12, dans laquelle la dose est dans la plage de 1 ng à 500 ng d'adénosine par jour.

14. Utilisation selon la revendication 12, dans laquelle les cellules précurseurs neuronales encapsulées comprennent d'environ 20 000 à environ 2 000 000 de cellules précurseurs neuronales déficientes en termes de production d'adénosine kinase et comprenant un défaut dans les deux allèles du gène d'adénosine kinase.

15. Utilisation d'une cellule précurseur neuronale selon la revendication 1, pour la préparation d'un médicament pour le traitement de l'épilepsie chez un sujet, dans laquelle les cellules précurseurs neuronales s'intègrent dans le cerveau et se différencient en cellules neuronales, dans laquelle les cellules neuronales différenciées libèrent une dose locale chronique d'une quantité thérapeutique d'adénosine, moyennant quoi ladite adénosine réduise l'activité épileptique chez le sujet, et dans laquelle ledit médicament doit être implanté.

16. Procédé de production d'une cellule précurseur neuronale déficiente en termes de production d'adénosine kinase et comprenant un défaut dans les deux allèles du gène d'adénosine kinase, comprenant :
(a) le traitement de cellules souches embryonnaires (ES) qui ont été isolées d'un mammifère, de façon à interrompre un allèle sur le gène d'adénosine kinase dans ladite cellule ES, dérivant ainsi une cellule ES comprenant un défaut dans un allèle d'un gène d'adénosine kinase ;
(b) le traitement desdites cellules ES dérivées de l'étape (a) de façon à inactiver l'allèle restant du gène d'adénosine kinase, générant ainsi une cellule ES comprenant un défaut dans les deux allèles du gène d'adénosine kinase ; et
la différenciation desdites cellules ES générées dans l'étape (b) en une cellule précurseur neuronale.
